(19) Europäisches Patentamt
European Patent Office
Office européen des brevets

(11) EP 0 434 979 B1

(12) **EUROPEAN PATENT SPECIFICATION**

(45) Date of publication and mention of the grant of the patent:
**03.01.1996 Bulletin 1996/01**

(51) Int Cl.[6]: **C07K 7/02**, A61K 38/08

(21) Application number: **90122330.5**

(22) Date of filing: **22.11.1990**

(54) **Peptide derivatives useful as antagonists of bombesin and gastrin-releasing peptide**

Peptidderivate, wertvoll als Antagonisten von Bombesin und Gastrin-freisetzendem Peptid

Dérivés peptidiques utiles comme antagonistes de la bombésine et du peptide libérant le gastrine

(84) Designated Contracting States:
**AT BE CH DE DK ES FR GB GR IT LI LU NL SE**

(30) Priority: **22.11.1989 US 440547**
**31.10.1990 US 603830**

(43) Date of publication of application:
**03.07.1991 Bulletin 1991/27**

(73) Proprietor:
**MERRELL DOW PHARMACEUTICALS INC.**
**Cincinnati, Ohio 45215 (US)**

(72) Inventors:
- **Edwards, Judson V.**
  **Cincinnati, Ohio 45231 (US)**
- **Fanger, Bradford O.**
  **Cincinnati, Ohio 45242 (US)**

(74) Representative: **VOSSIUS & PARTNER**
**D-81634 München (DE)**

(56) References cited:
**EP-A- 0 309 297**

- **BIOCHEMICAL & BIOPHYSICAL RESEARCH COMMUNICATIONS (BBRC), vol. 155, no. 1, 30th August 1988, pages 359-365; P.J. WOLL et al.: "[Leu13-psi(CH2NH)leu14]bombesin is a specific bombesin receptor antagonist in Swiss 3T3 cells"**
- **CHEMICAL ABSTRACTS, vol. 111, no. 11, 11th September 1989, page 96, abstract no. 90576e, Columbus, Ohio, US; A.F. SPATOLA et al.: "Comparing pseudopeptides: cyclic enkephalins with psi [CH2S], psi [CH2SO] and psi [CH2NH] amide bond surrogates", & COLLOQ. INSERM 1989, 174(FORUM PEPT., 2ND. 1988), 45-54**
- **CHEMICAL ABSTRACTS, vol. 109, no. 3, 18th July 1988, page 662, abstract 23372f, Columbus, Ohio, US; C.W. SMITH et al.: "Synthesis and renin inhibitory activity of angiotensinogen analogs having dehydrostatine, luepsi[CH3S]Val, or Luepsi[CH3SO] Val at the P2, P1 cleavage site", & J. MED. CHEM. 1988, 31(7), 1377-82**
- **CHEMICAL ABSTRACTS, vol. 105, no. 11, 15th September 1986, page 88, abstract no. 91511t, Columbus, Ohio, US; J.V. EDWARDS et al.: "In vitro activity profiles of cyclic and linear enkephalin pseudopeptide analogs", & BIOCHEM. BIOPHYS. RES. COMMUN. 1986, 136(2), 730-6**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

EP 0 434 979 B1

Printed by Jouve, 75001 PARIS (FR)

## Description

This invention relates to novel antimitotic and antisecretory peptides that are antagonists to the related peptides Bombesin and Gastrin Releasing Peptide which are useful in controlling; (1) the growth of small cell lung and prostatic carcinomas, and (2) gastric acid secretions, causative and symptomatic of peptic (esophageal, gastric, and duodenal) ulcers.

Bombesin is a 14 amino acid peptide, originally isolated from the skin of the frog Bombina bombina. Bombesin is known to have a range of effects including induction of gastric and pancreatic secretion, stimulation of the nervous system, induction of myoelectric and contractile activity of intestinal myocytes, reduction of renal blood flow, secretion of pituitary hormones, and growth promotion.

Gastrin Releasing Peptide (GRP), a 27 amino acid peptide, was isolated later in mammals, and found to have an identical carboxy terminus to bombesin, with analogous properties. Further, gastrin releasing peptide and the structurally related bombesin have been shown to compete for binding to high affinity receptors on small cell lung carcinoma cells (SCLC), and prostatic endothelial cells. Binding with any of these growth factors causes measurable [$^3$H]thymidine uptake and clonal growth.

Many studies have implicated cell growth factors in tumor cell growth by activation of growth factor receptors. In this regard, Bombesin/GRP and receptor activation is related to other well-known growth factors and oncogenes. There has been considerable interest in the design and development of competitive bombesin receptor antagonist as possible antimitotic agents since the discovery that these peptides, bombesin and GRP, act as potent autocrine growth factors in human small cell lung and possibly also prostatic carcinoma systems. Underlying support for this approach comes from experiments utilizing anti-bombesin monoclonal antibodies to prevent binding of its receptor and eliciting a mitogenic response. Experiments using monoclonal antipeptide antibodies demonstrated clonal growth inhibition of SCLC cells *in vitro* and in xenografts of nude mice *in vivo.* The mitogenic response begins with the production of intracellular signals by the ligand-receptor complex. For GRP/bombesin, this includes activation of a G-protein, which in turn activates phospholipase C (PLC). PLC converts phosphatidylinositol phosphate (PI) into inositol-1,4,5,-triphosphate ($IP_3$) and diacylglycerol (DAG). $IP_3$ and DAG are believed to be intracellular signals of the GRP/bombesin receptor pathway activation.

After Bombesin/GRP cell receptors were established on SCLC cells, receptors were also found to be present on human prostate cells. Subsequent studies also firmly established the growth effects of bombesin/GRP on prostatic epithelial cells in vitro utilizing bombesin/GRP antibodies. Further, cell proliferation studies of PC3 (a human prostatic carcinoma cell line) and PMU23 (a epithelial cell line from a primary culture of a stage III prostatic carcinoma) demonstrated a dose dependent Bombesin/GRP growth curve. These data suggest that there may be an autocrine or paracrine enhancement of tumor cell growth within the prostate gland. Therefore, interrupting the effect of GRP/bombesin may be useful for treating the progression of prostatic cancers where these factors are acting as autocrine or paracrine mitotic agents.

Bombesin and GRP are also capable of inducing gastric and pancreatic secretion and have demonstratable saturable receptors on cells of pancreatic (B-Cells) and intestinal (C-cells) origin, and therefore, antagonist of these receptors may serve in treatment of peptic ulcers and intestinal and pancreatic disorders, and may extend to adenocarcinomas of these tissues.

Studies with the anti-bombesin/GRP antibodies lead to the hypothesis that it may be possible to disrupt the autocrine growth cycle of bombesin/GRP using designed peptide receptor antagonists. Since then several types of Bombesin antagonist have been reported. These antagonist have been defined by type and position of the substitutions of the natural sequence. Early receptor antagonist suffered from low potency, lack of specificity, and toxicity which presented serious problems with scientific and therapeutic use.

More recent work has concentrated on modification of the carboxy terminal (C-terminal) region of these peptides to interrupt the receptor interaction utilizing a variety of different types of C-terminal modified analogs. These have included incorporation of D-amino acids, non-peptide bonds for example ($\Psi[CH_2NH]$), amide, and ester modifications. These alterations gave rise to certain peptides having improved characteristics.

EP-A 309 297 discloses a therapeutic peptide having a non-peptide bond instead of a peptide bond, which is an analogue to bombesin and is capable of acting as a competitive inhibitor of naturally occurring bombesin.

The applicants have prepared linear peptide analogs of the natural bombesin containing a non-peptide bond between amino acids 13 and 14, consisting of a thiomethylene group ($\Psi[CH_2S]$) to increase the conformational flexibility of the bond, and therefore the overall conformational flexibility of the peptide. The applicants have also prepared peptides containing the corresponding methylene sulfoxide ($\Psi[CH_2S(O)]$) by carrying out the appropriate oxidation of the thiomethylene. Oxidation of thiomethylene to the methylene sulfoxide affords two isomers. The applicants have physically separated the peptide isomers of the methylene sulfoxide, designated ($\Psi[CH_2S(O)]^I$) and ($\Psi[CH_2S(O)]^{II}$), and have tested those compounds in the appropriate assays. The applicants have demonstrated that the thiomethylene and methylene sulfoxide analogs of bombesin are *in vitro* antagonists of the natural molecule by (1) acting as competitive inhibitors of receptor binding, and (2) by antagonizing the biological actions of gastrin releasing peptide. The peptide antagonists

of this invention potentially possess significant antimitotic and/or antisecretory activity and therefore may allow for a scientifically interesting and therapeutically significant adjunct to cancer therapy and the treatment of ulcers. Moreover, the presence of thiomethylene or a methylene sulfoxide functionality may provide for enhanced potency and extended duration of action.

Claimed are peptide derivatives of the formula

$$X\text{-}N\text{-}A_1\text{-}A_2\text{-}A_3\text{-}A_4\text{-}A_5\text{-}A_6\text{-}A_7\text{-}A_8\text{-}A_9\text{-}A_{10}\text{-}A_{11}\text{-}A_{12}\text{-}A_{13}\text{-}\Psi\text{-}A_{14}\text{-}C\text{-}Y$$

| | |
|---|---|
| wherein X | is an amino terminal residue selected from hydrogen, one or two alkyl groups from 1 to 10 carbon atoms, one or two acyl groups from 2 to 10 carbon atoms, carbobenzyloxy or t-butyloxy carbonyl; unless the amino terminal amino acid is a cyclic derivative and thereby X is omitted, |
| N | is a bond, unless the amino terminal amino acid is a cyclic derivative and thereby N is omitted, |
| $A_1$ | is pGlu or a bond |
| $A_2$ | is Gln or a bond |
| $A_3$ | is Arg or a bond |
| $A_4$ | is Leu or a bond |
| $A_5$ | is Gly or a bond |
| $A_6$ | is Asn, or D-Phe; |
| $A_7$ | is Gln or Eis; |
| $A_8$ | is Trp; |
| $A_9$ | is Ala; |
| $A_{10}$ | is Val; |
| $A_{11}$ | is Gly or D-Ala; |
| $A_{12}$ | is Eis; |
| $A_{13}$ | is Phe or Leu; |
| $\Psi$ | is a dipeptide determinant of $A_{13}$aa $\Psi$ $A_{14}$aa where $\Psi$ is $[CH_2S]$ , $[CH_2S(O)]$, $\Psi[CH_2S(O)]^{I}$ or $\Psi$ $[CH_2SO]^{II}$ where in $A_{13}$aa and $A_{14}$aa designate the substituent amino acids; |
| A14 | is Leu, Nle or Met; |
| C | is a bond; |
| Y | is a carboxy terminal residue selected from OH, ($C_1$-$C_8$) alkoxyester, amino, mono or di ($C_1$-$C_4$) alkyl amide ($C_1$-$C_4$)alkylanine, ($C_l$-$C_4$)thioalkylether, or pharmaceutically acceptable salt thereof. |

The following common abbreviations of; (1) amino acids and their three letter codes, (2) modified and unusual amino acids, and (3) terminal amino and carboxy substituents used throughout this specification:

**(1): THE AMINO ACIDS AND THEIR THREE LETTER CODE**

| L-AMINO ACIDS | D-AMINO ACIDS |
|---|---|
| Ala - alanine | D-Ala - D-alanine |
| Arg - arginine | D-Arg - D-arginine |
| Asn - asparagine | D-Asn - D-asparagine acid |
| Cys - cysteine | D-Cys - D-cysteine |
| Gly - glycine | |
| Glu - glutamic acid | D-Glu - D-glutamic acid |
| Val - valine | D-Val - D-valine |
| Gln - glutamine | D-Gln - D-glutamine |
| His - histidine | D-His - D-histidine |
| Ile - isoleucine | D-Ile - D-isoleucine |
| Leu - leucine | D-Leu - D-leucine |
| Lys - lysine | D-Lys - D-lysine |
| Phe - phenylalanine | D-Phe - D-phenyl-alanine |
| Met - methionine | D-Met - D-methionine |
| Pro - proline | D-Pro - D-proline |
| Ser - serine | D-Ser - D-serine |
| Thr - threonine | D-Thr - D-threonine |
| Trp - tryptophan | D-Trp - D-tryptophan |
| Tyr - tyrosine | D-Tyr - D-tyrosine |

**(2): MODIFIED AND UNUSUAL AMINO ACIDS**

pGlu - pyroglutamic acid

Nle - norleucine

**(3): TERMINAL AMINO AND CARBOXY SUBSTITUENTS**

Ac - acetyl

Azt - azetidine-2-carboxylate

Cin - cinnamoyl

DhCin - 3,4-dihydrocinnamoyl

Glt - glytaryl

Mal - maleyl

Oac - 8-aminooctanoic acid

Oct - n-octane

Suc - succinyl

Tfa - trifluoroacetyl

# - C-terminal amide

The following designates the known naturally occurring amino acid sequence variations of GRP AND BOMBESIN:

**SEQUENCE IDENTIFICATION**

AMINO ACID SEQUENCE VARIATIONS OF GRP AND BOMBESIN

GRP:

| | | | | | | | | | | | | | | |
|---|---|---|---|---|---|---|---|---|---|---|---|---|---|---|
| 1 | | | | 5 | | | | | 10 | | | | | 15 |
| Val | Pro | Leu | Pro | Ala | Gly | Gly | Gly | Thr | Val | Leu | Thr | Lys | Met | Tyr |
| Ala | | Val | Ser | Val | | Gln | Ser | Pro | Ala | | Ala | | Ile | Thr |
| | | | Gln | Pro | | | | | | | Asp | | | |
| | | | | Gly | | | | | | | | | | |

| | | | | | | | | | | | | |
|---|---|---|---|---|---|---|---|---|---|---|---|---|
| | | | | 20 | | | | | 25 | | | |
| Pro | Arg | Gly | Asn | His | Trp | Ala | Val | Gly | His | Leu | Met-$NH_2$ | (Human) |
| | | | Ser | | | | | | | | | |

BOMBESIN:

| | | | | | | | | | | | | | |
|---|---|---|---|---|---|---|---|---|---|---|---|---|---|
| 1 | | | | 5 | | | | | 10 | | | | |
| pGlu | Gln | Arg | Leu | Gly | Asn | Gln | Trp | Ala | Val | Gly | His | Leu | Met-$NH_2$ |
| | | | | | | | | | | | | | (Human) |

LITORIN:

| | | | | | | | | |
|---|---|---|---|---|---|---|---|---|
| 1 | | | | 5 | | | | 10 |
| pGlu | Gln | Trp | Ala | Val | Gly | His | Phe | Met-$NH_2$ |

Amino Acids & Modifications

As is customary, the structure of peptides written out herein is such that the amino terminal end is on the left side of the chain and the carboxy terminal end is on the right side of the chain.

The naturally occuring amino acids, with the exception of glycine, contain a chiral carbon atom. Unless otherwise specifically indicated, the optically active amino acids, referred to herein, are of the L-configuration. For example, any of the amino acids of the $A_1$ or $A_{14}$ group can be of the D- or L-configuration. Furthermore, it is understood that after oxidation of thiomethylene to the methylene sulfoxide two peptide isomers of the methylene sulfoxide exist, designated

($\Psi[CH_2S(O)]^I$) and ($\Psi[CH_2S(O)]^{II}$). Furthermore it is understood that these isomers can be isolated and their absolute configuration determined. The term $\Psi[CH_2S(O)]$ used herein is used to refer to the existence of both isomers either jointly or separately.

An alkyl group and the alkyl portion of an alkoxy group is taken to include straight, branched, or cyclic alkyl groups, for example, methyl, ethyl, propyl, isopropyl, butyl, isobutyl, tert-butyl, pentyl, isopentyl, sec-pentyl, cyclopentyl, hexyl, isohexyl, cyclohexyl and cyclopentylmethyl, heptyl, octyl(Oct), 8-aminooctanoic acid(Oac). An acyl group of from 2 to 10 carbon atoms is taken to include straight, branched, cyclic, saturated and unsaturated acyl groups having 1 or 2 carbonyl moieties per group, for example, acetyl(Ac), azetidine-2-carboxylate(Azt), benzoyl succinyl, cinnamoyl(Cin), 3,4-dihydrocinnamoyl(DhCin), maleyl(Mal), and glutaryl(Glt). Both alkyl and acyl substituents are taken to include those groups with halogen substituents, where a halogen group is a fluoro, chloro, bromo or iodo, for example, trifloroacetyl (Tfa). Cyclic derivatives of N-terminal amino acid residues include pyroglutamic acid (pGlu) and homoserine lactone (Hse).

Groups of α-amino acids can be defined by certain charge characteristics. There are two general characteristics of side chains: nonpolar and polar. The nonpolar residues are made up of the hydrophobic residues which includes those with aliphatic hydrocarbon side chains: Gly, Ala, Val, Leu, Ile, Nle, Pro and the aromatic group Phe and Trp, and the pseudohydrocarbon, Met. The polar amino acids are made up three groups: (1) The acidic hydrophilic residues, (2) the neutral residues, and (3) basic hydrophilic residues. The neutral residues contain the hydroxyl-containing residues, Ser and Thr; the amides, Asn and Gln; aromatic rings, Tyr and Trp; the sulfhydryl groups, Cys, and small structurally accomodating amino acids Ala and Gly . In the polar class are "Acidic hydrophilic" residues that include aspartic acid, glutamic acid, and tyrosine and the "basic hydrophilic" residues that include His, Lys, and Arg.

Y designates the chemical group(s) that may be utilized to substitute or modify the terminal amino acid. Therefore, Y may be a carboxamide, alkoxy ester, alkylamide, alkyl amine, or thioalkylether.

The polypeptides of formula 1 can form pharmaceutically acceptable salts with any non-toxic, organic or inorganic acid. Illustrative inorganic acids which form suitable salts include hydrochloric, hydrobromic, sulphuric and phosphoric acid and acid metal salts such as sodium monohydrogen orthophosphate and potassium hydrogen sulfate. Illustrative organic acids which form suitable salts include the mono, di and tricarboxylic acids. Illustrative of such acids are, for example, acetic, glycolic, lactic, pyruvic, malonic, succinic, glutaric, fumaric, malic, tartaric, citric, ascorbic, maleic, hydroxymaleic, benzoic, hydroxybenzoic, phenylacetic, cinnamic, salicylic, 2-phenoxybenzoic and sulfonic acids such as methane sulfonic acid and 2-hydroxyethane sulfonic acid. Salts of the carboxy terminal amino acid moiety include the non-toxic carboxylic acid salts formed with any suitable inorganic or organic bases. Illustratively, these salts include those of alkali metals, as for example, sodium and potassium; alkaline earth metals, such as calcium and magnesium; light metals of Group IIIA including aluminum; and organic primary, secondary and tertiary amines, as for example, trialkylamines, including triethylamine, procaine, dibenzylamine, 1-ethenamine, N,N′-dibenzylethylenediamine, dihydroabietylamine, N-(lower)alkylpiperidine, and any other suitable amine.

Utilizing conventional nomenclature employed by peptide chemists, wherein $A_{13}$-Ψ-$A_{14}$ are those compounds wherein the moiety connecting the $A_{13}$ residue to the $A_{14}$ residue is comprised of two Leu residues linked by a reduced thiomethylene bond and can be designated as PheΨ$[CH_2S]$Leu. This designation indicates that the carbonyl group of the penultimate Phe is reduced to a thiomethylene group. The procedure to prepare those peptide derivatives of formula 1 wherein Ψ is a $-CH_2S-$ group, that is the $\Psi[CH_2S]$ compounds, is known from Spatola and Darlak, Tetrahedron Letters 44(3), 821-33 (1988). Other nomenclature designations used to describe the peptide derivatives of this invention are for methylene sulfoxides $\Psi[CH_2S(O)]$ (Edward and Spatola, Journal of Liquid Chromatography 915, 903-919 (1986)). Furthermore, after oxidation of thiomethylene to the methylene sulfoxide two peptide isomers of the methylene sulfoxide exist, designated ($\Psi[CH_2S(O)]^I$) and ($\Psi[CH_2S(O)]^{II}$), It is also understood that these isomers can be isolated and their absolute configuration determined. The term $\Psi[CH_2S(O)]$ is used to refer to the existance of both isomers either jointly or separately. ($\Psi[CH_2C(O)]^I$) and ($\Psi[CH_2S(O)]^{II}$) are characterized has having retention times of 5.40 minutes and 6.28 minutes respectively on a C18 column (Vydac analytical column; 4.6mm ID x 25cm, 5 micron C18) with the mobile phase pumped at a flow rate of 2 ml/minute with a linear gradient of 25% to 75% acetonitrile in water containing 0.1% trifluoroacetic acid over 25 minutes.

The ability of the peptide derivatives of this invention to act as antagonists of Bombesin/GRP can be demonstrated by the ability of such peptides to compete with radioiodinated bombesin/GRP for mammalian bombesin/GRP receptors using the method of Buck et al., Science 226: 987-989, 1984, and by the ability of such compounds to stimulate or to inhibit bombesin/GRP induced phosphatidylinositol turnover using the method of Bristow et al., British J. Pharmacol, 90: 211-21, 1987.

As with any generic group of chemical compounds, certain groups are preferred. Applicants prefer those peptide derivatives of formula 1 wherein the peptide is pGlu-Gln-Arg-Leu-Gly-Asn-Gln-Trp-Ala-Val-Gly-His-PheΨ$[CH_2S]$Leu-$NH_2$ , Ac-D-Phe-Gln-Trp-Ala-Val-D-Ala-His-PheΨ$[CH_2S]$Leu-$NH_2$, Asn-Gln-Trp-Ala-Val-Gly-His-Phe Ψ$[CH_2S]$Leu-$NH_2$, ASn-Gln-Trp-Ala-Val-Gly-His-Phe Ψ$[CH_2S(O)]$Leu-$NH_2$, pGlu-Gln-Trp-Ala-Val-Gly-His-Phe-Ψ$[CH_2S(O)]$Leu-$NH_2$, pGlu-Gln-Trp-Ala-Val-Gly- His-Phe Ψ$(CH_2S]$Nle-$NH_2$, pGlu-Gln-Trp-Ala-Val-Gly-His-Phe Ψ$(CH_2S(O)]^I$Leu-$NH_2$,

pGlu-Gln-Trp-Ala-Val-Gly-His-PheΨ[$CH_2$S]Leu-$NH_2$ or pGlu-Gln-Trp-Ala-Val-Gly-His-PheΨ[$CH_2$S(O)]$^{II}$Leu- $NH_2$.

Peptide Synthesis:

The peptides of this invention can be prepared by a variety of procedures readily known to those skilled in the art. Such procedures include the solid phase sequential and block synthesis, gene cloning and combinations of these techniques. The solid phase sequential procedure can be performed using established automated methods such as by use of an automated peptide sythesizer. In this procedure, the peptides were synthesized on the resin beginning with a protected dipeptide containing a inter-amino acid thiomethylene methylene sulfoxide bridge with the C-terminal amino acid attached to a methylbenzhydrylamine resin. The extension of the peptide sequence was done using standard methodology and that of the manufacturer and that known by people skilled in the art.

After completion of coupling of the sequence either the Boc protecting group was left in place or it was removed and the N-terminal amino group acylated. Displacement of the protected fragment from the resin was accomplished using the hydrogen fluoride procedure.

The α-amino protecting group employed with each amino acid introduced into the polypeptide sequence may be any such protecting group known to the art. Among the classes of α-amino protecting groups contemplated are (1) acyl type protecting groups such as: formyl, trifluoroacetyl, phthalyl, toluenesulfonyl (tosyl), benzenesulfonyl, nitrophenylsulfenyl, tritylsulfenyl, o-nitrophenoxyacetyl and α-chlorobutyryl; (2) aromatic urethan type protecting groups such as benzyloxycarbonyl and substituted benzyloxycarbonyl, such as p-chlorobenzyloxycarbonyl, p-nitrobenzylcarbonyl, p-bromobenzyloxycarbonyl, p-methoxybenzyloxycarbonyl, 1-(p-biphenylyl)-1-methylethoxycarbonyl, α, α-dimethyl-3,5-dimethoxybenzyloxycarbonyl and benzhydryloxycarbonyl; (3) aliphatic urethan protecting groups such as tert-butyloxycarbonyl (Boc), diisopropylmethoxycarbonyl, isopropyloxycarbonyl, ethoxycarbonyl and allyloxycarbonyl; (4) cycloalkyl urethane type protecting groups such as cyclopentyloxycarbonyl, adamantyloxycarbonyl and cyclohexyloxycarbonyl; (5) thiourethane type protecting groups such as phenylthiocarbonyl; (6) alkyl type protecting groups such as triphenylmethyl (trityl) and benzyl; and (7) trialkylsilane groups such as trimethylsilane. The preferred α-amino protecting group is tert-butyloxycarbonyl (Boc).

As is known in the art of solid phase peptide synthesis many of the amino acids bear functionalities requiring protection during the chain preparation. The use and selection of the appropriate protecting group is within the ability of those skilled in the art and will depend upon the amino acid to be protected and the presence of other protected amino acid residues on the peptide. The selection of such a side chain protecting group is critical in that it must be one which is not removed by cleavage during cleavage of the protecting group of the α-amino moiety. For example, suitable side chain protecting groups for lysine are benzyloxycarbonyl and substituted benzyloxycarbonyl, said substituent being selected from halo (e.g., chloro, bromo, fluoro) and nitro (e.g., 2-chlorobenzyloxycarbonyl, p-nitrobenzyloxycarbonyl, 3,4-dichlorobenzyloxycarbonyl), tosyl, t-amyloxycarbonyl, t-butyloxycarbonyl and diisopropylmethoxycarbonyl. The alcoholic hydroxyl group of threonine and serine can be protected with an acetyl, benzoyl, tert-butyl, trityl, benzyl, 2,6-dichlorobenzyl or benzyloxycarbonyl group. The preferred protecting group is benzyl.

The selection of an appropriate coupling reagent is within the skill of the art. Particularly suitable coupling reagents where the amino acid to be added is Gln, Asn or Arg are N,N′-diisopropylcarbodiimide and 1-hydroxybenzotriazole. The use of these reagents prevents nitrile and lactam formation. Other coupling agents are (1) carbodiimides (e.g., N,N′-dicyclohexylcarbodiimide and N-ethyl-N'-(γ-dimethylaminopropylcarbodiimide)); (2) cyanamides (e.g., N,N-dibenzylcyanamide); (3) ketenimines; (4) isoxazolium salts (e.g., N-ethyl-5-phenyl-isoxazolium-3'-sulfonate; (5) monocyclic nitrogen containing heterocyclic amides of aromatic character containing one through four nitrogens in the ring such as imidazolides, pyrazolides, and 1,2,4-triazolides. Specific heterocyclic amides that are useful include N,N′-carbonyldiimidazole and N,N-carbonyl-di-1,2,4-triazole; (6) alkoxylated acetylene (e.g., ethoxyacetylene); (7) reagents which form a mixed anhydride with the carboxyl moiety of the amino acid (e.g., ethylchloroformate and isobutylchloroformate) or the symmetrical anhydride of the amino acid to be coupled (e.g., Boc-Ala-O-Ala-Boc), (8) nitrogen containing heterocyclic compounds having a hydroxy group on one ring nitrogen (e.g., N-hydroxyphthalimide, N-hydroxysuccinimide and 1-hydroxybenzotriazole), and (9) diphenyl phosphorylazide. Other activating reagents and their use in peptide coupling are described by Kapoor, J. Pharm. Sci. 59, pp. 1-27 (1970). Applicants prefer the use of the symmetrical anhydride as a coupling reagent for all amino acids except Arg, Asn and Gln.

Each protected amino acid or amino acid sequence is introduced into the solid phase reactor in about a four-fold excess and the coupling is carried out in a medium of dimethylformamide: methylene chloride (1:1) or in dimethylformamide alone or preferably methylene chloride alone. In cases where incomplete coupling occurs, the coupling procedure is repeated before removal of the α-amino protecting group, prior to the coupling of the next amino acid in the solid phase reactor. The success of the coupling reaction at each stage of the synthesis is monitored by the ninhydrin reaction as described by E. Kaiser et al, Analyt. Biochem. 34, 595 (1970).

Following the coupling of the α-amino protected amino acid to the resin support, the protecting group is removed using any suitable procedure as by using trifluoroacetic acid in methylene chloride, trifluoroacetic acid alone, or HCl in

dioxane. The deprotection is carried out at a temperature of between 0°C and room temperature. Other standard cleaving reagents and conditions for removal of specific $\alpha$-amino protecting groups may be used. After removal of the $\alpha$-amino protecting group the other amino protected amino acids are coupled step-wise in the desired order. Alternatively, multiple amino acid groups may be coupled by the solution method prior to coupling with the resin supported amino acid sequence.

After the desired amino acid sequence has been obtained, the peptide is removed from the resin. This can be done by hydrolysis such as by treatment of the resin bound polypeptide with an amino acid alcohol and acetic acid in dichloromethane (DCM). Protecting groups can be removed by procedures well known in the art. Typically protecting group removal is done after the peptide chain synthesis is complete but the protecting groups can be removed at any other appropriate time. Purification of peptides is principally accomplished through preparative reversed phase high performance liquid chromatography and those techniques known to those skilled in the art.

Alternatively, if the methylene sulfoxide is to be prepared the oxidation of the thiomethylene can be done by a number of oxidizing reagents. One method to accomplish the desired oxidation is to treat the thiomethylene peptide with a aqueous solution containing hydrogen peroxide to convert the peptide to its methylene sulfoxide. Isolation of the isomers can be accomplished by high pressure liquid chromatography (HPLC) as known and used by those skilled in the art. For example C18 reverse phase HPLC can be suitably adapted for the isolation of the methylene sulfoxide peptide isomers.

The natural history of peptic ulcer disease is one of recurrent exacerbations and remissions. As result, ulcerative diseases should be treated as a chronic disorder. Peptic (esophageal, gastric, and duodenal) ulcers occur in areas of the gastrointestinal tract exposed to acid and pepsin. The compounds of the present invention, which are useful in the treatment of gastrointestinal and/or pancreatic ulcers and may be effective in resultant hypersecretions occurring from the pancrease and/or stomach, particularly hydrochloric acid and pepsin. As such compounds of this invention may serve as an appropriate intervention to treat peptic ulcers.

The appropriate dose of a peptide derivative of this invention when used in the treatment of peptic ulcers is from 0.2 mg/kg to 250 mg/kg of patient body weight per day depending on factors such as the patient, the severity of the peptic ulcer to be treated and the peptide derivative selected. The suitable dose for a particular patient can be readily determined. Preferably from 1 to 4 daily doses would be administered typically with from 5 mg to 100 mg of active compound per dose. The amount of a peptide of this invention required to inhibit gastric acid secretion can be readily determined by those skilled in the art.

Use of bombesin/GRP analogs in cancer therapy is indicated for the treatment of SCLC and prostatic carcinomas and prevention of a variety of other cancer conditions. Those experienced in this field are readily aware of the circumstances requiring cancer therapy. The term "patient" used herein is taken to mean mammals such as primates, including humans, sheep, horses, cattle, pigs, dogs, cats, rats and mice.

As used herein, the term "tumor tissue" means both benign and malignant tumors or neoplasms and includes melanomas, lymphomas, leukemias, and sarcomas. Illustrative examples of tumor tissues are cutaneous such as malignant melanomas and mycosis fungoides; hematologic tumors such as leukemias, for example, acute lymphoblastic, acute myelocytic, or chronic myelocytic leukemia; lymphomas such as Hodgkin's disease or malignant lymphoma; gynecologic tumors such as ovarian and uterine tumors; urologic tumors such as those of the prostate, bladder, or testis; soft tissue sarcomas, osseus, or non-osseous sarcomas, breast tumors; tumors of the pituitary, thyroid, and adrenal cortex; gastrointestinal tumors such as those of the esophagus, stomach, intestine, and colon; pancreatic and hepatic tumors; laryngeae papillomatosis and lung tumors.

The term "controlling the growth" and the concept of treating a cancer means slowing, interrupting, arresting, or stopping the growth and metastases of a rapidly proliferating tumor in a warm blooded animal; it being understood that treatment in a warm blooded animal does not generally provide a "cure" for the tumor in the sense that necessarily the tumor tissue is destroyed or totally eliminated.

The appropriate dose of an peptide derivative of this invention when used in the treatment of, or controlling the growth of a cancer or tumor tissue, including small cell lung carcinomus or prostatic cancers is from 0.2 mg/kg to 250 mg/kg of patient body weight per day depending on factors such as the patient, the severity of the small cell lung carcinoma to be treated and the peptide derivatives selected. The suitable dose for a particular patient can be readily determined. Preferably from 1 to 4 daily doses would be administered typically with from 5 mg to 100 mg of active compound per dose. The amount of a peptide of this invention required to inhibit SCLC growth can be readily determined by those skilled in the art.

It is generally known that therapeutic agents used in the treatment of cancer can be used in conjunction with other therapeutic agents or therapies known to be useful in the treatment of cancer. The peptides of invention can also be used in conjunctive therapy. For example, a peptide of structure 1-5 can be administered in conjunction with surgical excision of the tumor or with radiation therapy, immunotherapy, or local heat therapy. In the preferred mode of treating cancer the peptide of structure 1-5 are administered in conjunction with a chemical cytotoxic agent known to be useful for tumor therapy. Illustrative chemical cytotoxic agents are cyclophosphamide, methotrexate, prednisone, 6-mercaptopurine, procarbazine, danorubicin, chlorambucil, cytosine arabinoside, 6-thioguanine, thio TEPA, 5-fluorouracil, 5-fluoro-2-deoxyuridine, 5-azacytidine, nitrogen mustard, 1,3-bis(2-chloroethyl)-1-nitrosourea (BCNU), 1-(2-chloroethyl) -3-cyclohexyl-1-nitrosourea (CCNU), busulfan, adriamycin, bleomycin, vindesine, cycloeucine, or methylglyoxal bis(guanylhydrazone) (MGBG). When such combination therapy is employed, the effect of the cytotoxic agent may be potentiated. The remission produced by the cytotoxic agent may be enhanced and regrowth of the tumor may be slowed or prevented. Use of such combination therapy therefore allows for smaller doses or fewer individual doses of the cytotoxic agent to be employed. The term "combination therapy" contemplates the administration of a peptide of structure 1-5 immediately prior to the beginning of therapy with a cytotoxic agent, concurrently with such therapy, or during the time immediately following the cessation of such therapy. When such combination therapy is employed for the treatment of a tumor the cytotoxic agent may be administered at a dosage known in the art to be effective for treating the tumor. However, a peptide of structure 1-5 may produce an additive or synergistic effect with a cyctotoxic agent against a particular tumor. Thus when such combination antitumor therapy is used, the dosage of the cytotoxic agent administered may be less than that administered when the cytotoxic agent is used alone. In combination with a peptide derivative of structure 1-5, the cytotoxic agent may, therefore, be administered at a lower dosage or at less frequent intervals as compared to the cytotoxic agent used alone. Similarly when combination therapy is used, the dosage or frequency of administration of a peptide of structure 1-5 may be less than when used without a cytotoxic agent.

Although some of the peptide derivatives may survive passage through the gut following oral administration, applicants prefer non-oral administration, for example, subcutaneous, intravenous, intramuscular or intraperitoneal; administration by depot injection; by implant preparation; or by application to the mucous membranes, such as, that of the nose, throat and bronchial tubes, for example, in an aerosol can containing a peptide derivative of this invention in a spray or dry powder form.

For parenteral administration the compounds may be administered as injectable dosages of a solution or suspension of the compound in a physiologically acceptable diluent with a pharmaceutical carrier which can be a sterile liquid such as water and oils with or without the addition of a surfactant and other pharmaceutically acceptable adjuvants. Illustrative of oils which can be employed in these preparations are those of petroleum, animal, vegetable, or synthetic origin, for example, peanut oil, soybean oil, and mineral oil. In general, water, saline, aqueous dextrose and related sugar solutions, ethanol and glycols such as propylene glycol or polyethylene glycol are preferred liquid carriers, particularly for injectable solutions.

This invention is illustrated by the following, nonlimiting examples.

## EXAMPLE 1

Preparation of Dipeptide Determinant $A_{13}aa\Psi[CH2S]A_{14}aa$ 2-Mercapto-4-Methylpentanoic Acid (Mercapto-Leucine; Compound 1)

A solution of D-Leucine (5g) and potassium bromide (114g) in 400 ml of 2.5 N $H_2SO_4$ was cooled to -5°C in an ice salt bath. A cold solution of $NaNO_2$ (30g/70ml water, O-5°C) was added dropwise with stirring. The reaction was allowed to proceed for - 14 hrs at room temperature. The reaction was then extracted with 75ml portions of ether three times. The ether extract was dried over anhydrous sodium sulfate. The solution was filtered and the ether was evaporated. The resulting clear oil, 2-bromo-4-methyl-pentanoic acid (Martin and Greco, (1968) J. Org. Chem. 33, 1275-1276) (18g) was committed to a 250ml solution of 33% sodium trithiocarbonate with stirring at 0°C. The reaction was stirred for 48 hrs and then acidified at 0°C with judicious addition of 10N $H_2SO_4$. The acidified solution was then extracted with 75ml portions of ether three times. The ether extracts were dried over anhydrous sodium sulfate, the ether was removed *in vacuo,* and the resulting yellowish oil (17g) was vacuum distilled. The final yield was 15.3g of (S)-2-mercapto-4-methylpentanoic acid; b.p. 92-93°C (0.75mmHg); $[\alpha]_D25$=-23.2 (cl,MeOH).

### (S)-(tert-Butyloxycarbonyl)-2-Amino-3-Phenyl-Propanyl-p-Toluenesulfonate (Compound 2)

The starting reagent for the title compound, was synthesized from (S)-(tert-Butyloxycarbonyl)-2-Amino-3-Phenyl-Propanol(4.5g, 0.0179moles; prepared from L-phenylalaninol (Sigma) and di-tert-butyldicarbonate). The starting reagent was then added to 20 mls of pyridine under anhydrous conditions and chilled to -40°C in a dry ice/acetone bath. To the mixture tosyl chloride was then added (6.9g, 3.6mmol). The reaction mixture was then run at 4°C. No effort was made to remove accumulating deposits of pyridinium chloride. Upon termination of the reaction, the pyridine was removed *in vacuo,* and the resulting solid was taken up in ether. The ether extract was dried over anhydrous sodium sulfate, filtered, and the ether removed *in vacuo*; yielding 10.5g of a oil. Crystals of the product were obtained from percipitation of the oil in ethyl acetate and hexane; yielding 9.0g of a white solid; m.p. 109-110°C.

(S)-(S)-tert-Butyloxcarbonyl-PheΨ[CH2S]Leu-OH (Compound 3)

A 0.43 M solution (Solution A) of sodium ethoxide was prepared with freshly cut sodium and anhydrous ethanol. An ethanol solution (solution B) of (S)-2-mercapto-4-methylpentanoic acid, Compound 1 (0.72g in 25 mls), was prepared. A 13.5ml volume of solution A was slowly added to 15ml of solution B under nitrogen atmosphere. The solution was stirred for five minutes, and the ethanol removed *in vacuo,* and the white solid repeatedly evaporated with benzene until dry. The resulting disodium salt of mercaptoleucine was dissolved in ~1ml of dimethylsulfoxide (DMSO) to which was added 1.58g of compound 2 dissolved in 2mls of DMSO, and stirred overnight. The reaction mixture was combined with 175 ml of distilled water and extracted with 20ml portions of ether three times and then acidified with 5N HCl with stirring at 0°C. The aqueous solution was re-extracted 3X with ethyl acetate. The extract was washed with a saturated NaCl solution and dried over sodium sulfate, filtered, and the ethyl acetate removed *in vacuo* yielding 1.05g of a clear oil. This was crystallized from ethyl acetate and hexane; yielding a white solid; (0.83g), (mp, 110-111°C), [α]25= 52.5 (CO.88 1, MeOH)).

[$Phe_{13}$Ψ[$CH_2$S]$Leu_{14}$]Bombesin (Compound 4)

Acylation of compound 3 (0.20g) to 4-methylbenzhydrylamine (MBHA) resin was accomplished by combining Compound 3, dicyclohexylcarbodiimide (0,113g), hydroxybenzotriazole (0.074g) and MBHA (0.414g) in 20ml methylene chloride: DMF (9:1;v:v). Coupling of compound 3 was perfomed on a Vega solid phase peptide synthesizer placed in a semiautomated mode according to the literature procedure. Following a 2 hr coupling the reaction was monitored with ninhydrin. The resulting Boc-PheΨ[$CH_2$S]Leu MBHA resin amide was washed with DMF and ethanol and dried, yielding 0.498g corresponding to a substitution level of 0.53 mmol/g resin. The solid phase peptide synthesis for elongation of the amino acid sequence was performed on an Applied Biosystems peptide synthesizer using standard methodology, that of the manufacture, and that known by people skilled in the art.

The completed resin bound peptide (0.712g yield) was cleaved from the resin employing hydrogen fluoride (10ml/g resin) at 0°C in the presence of anisole (ethanedithiol; 1ml:.5 mg of resin) for 1 hr. Following, removal of the HF the resin was stirred and extracted with diethyl ether (2x30ml) and extracted with 30% acetic acid. Lyphilization afforded 250 mg of crude product. A portion of the product (400mg) was purified on preparative reverse phase high performance liquid chromatography with a C18 Dynamax column employing a mobile phase gradient elution (15 min acetonitrile gradient 20-30% at 40ml/min; established from reservoirs of acetonitrile and 0.1 %TFA in water). Four fractions of the principle peak were collected monitoring absorbance of the compound at $A_{214}$.

The peptides obtained by this method gave the desired molecular ion peak by FAB-MS and had an amino acid analysis in accordance with the desired peptide. In this way the following peptides having the stated properties were prepared.

1) pGlu Gln Arg Leu Gly Asn Gln Trp Ala Val Gly His PheΨ[$CH_2$S]Leu-$NH_2$
MW 1637 FAB-MS (MH+) 1638 $t_R$
55% peptide content

2) Ac-D-Phe Gln Trp Ala Val D-Ala His PheΨ[$CH_2$S]Leu-$NH_2$
MW 1160 FAB-MS (MH+) 1161 $t_R$
89% peptide content

3) Asn Gln Trp Ala Val Gly His PheΨ[$CH_2$S]Leu-$NH_2$
MW 1089 FAB-MS (MH+) 1090 $t_R$
52% peptide content

4) Asn Gln Trp Ala Val Gly His Phe$\Psi$[$CH_2S(O)$]Leu-$NH_2$
MW 1072 FAB-MS (MH+) 1073 $t_R$
50% peptide content

5) pGlu Gln Trp Ala Val Gly His Phe$\Psi$[$CH_2S(O)$]Leu-$NH_2$
MW 1069 FAB-MS (MH+) 1070 $t_R$
80% peptide content

6) pGlu Gln Trp Ala Val Gly His Phe$\Psi$[$CH_2S$]Nle-$NH_2$
MW 1069 FAB-MS (MH+) 1070 $t_R$

6a) pGlu Gln Trp Ala Val Gly His Phe$\Psi$[$CH_2S$]Leu-$NH_2$
MW 1069 FAB-MS (MH+) 1070 $t_R$
80% peptide content

Example 2

Methylene Sulfoxide Isomers: [$Phe_8\Psi[CH_2S(O)]Leu_9$]$I_{Litorin}$ & [$PHe_8\Psi[CH_2S(O)]Leu_9$]$II_{Litorin}$

A 3 ml solution of [$Phe_8[CH_2S]Leu_9$]Litorin (20mg) was prepared in the HPLC eluant solvent (0.1% TFA:acetonitrile, 75:25, v:v). To this sample solution was added 0.25 ml of a 5% solution of hydrogen peroxide. The sample was allowed to stand at 0°C for 2 hours whereupon it was injected onto a preparative reverse-phase system (C18 Dynamax 300A° column). Oxidation of the peptide to its diasteromeric methylene sulfoxides was monitored by high Pressure liquid chromatography (HPLC) by the appearance of two closely eluting peaks having retention times considerably earlier than that of the $CH_2S$ containing peptide. The methylene sulfoxides are accordingly separated and purfied by reverse-phase HPLC. Designation of Phe$\Psi$[$CH_2S(O)$]$^I$Leu Litorin and Phe$\Psi$[$CH_2S(O)$]$^{II}$Leu Litorin is based on their retention times by reverse phase HPLC.

The peptides obtained by this method gave the desired molecular ion peak by FAB-MS and had an amino acid analysis in accordance with the desired peptide. In this way the following peptides having the stated properties were prepared.

7) pGlu Gln Trp Ala Val Gly His Phe$\Psi$[$CH_2S(O)$]$^I$Leu-$NH_2$
MW 1085 FAB-MS (MH+) 1086 $t_R$
55% peptide content
Retention time 5.40 minutes*

8) pGlu Gln Trp Ala Val Gly His PheΨ[$CH_2S(O)$]$^{II}$Leu-$NH_2$
MW 1085 FAB-MS (MH+) 1086 $t_R$
63% peptide content
Retention time 6.28 minutes*

* Retention time based on retention on a Vydac analytical column; 4.6mmID x 25cm, 5 micron C18; flow rate of 2 ml/minute; linear gradient of 25% to 75% acetonitrile in water containing 0.1% trifluoroacetic acid over 25 minutes.

EXAMPLE 3

ANTAGONISM OF THE BOMBESIN RECEPTOR $Phe_{13}$ Ψ[$CH_2S$]$Leu_{14}$ BOMBESIN AS DEMONSTRATED BY COMPETITION AT THE RECEPTOR FOR IODINATED GRP

The pancreata from one or more mice were pooled, minced, and homogenized in 50 mM HEPES (pH 7.4) containing 120 mM NaCl and 5 mM KCl at 4°C and centrifuged at 37,500 X g for 15 minutes. The pellet was resuspended in 50 mM HEPES (pH 7.4) containing 10 mM EDTA and 300 mM KCl and incubated for 30 minutes at 4°C. The suspension was centrifuged as above and the pellet was washed two times in 50 mM HEPES (pH 7.4) containing protease inhibitors (10 μM phenylmethylsulfonyl fluoride, 1 μM thiorphan, 40 μg/ml bacitracin, 10mM iodoacetimide and 4 μg/ml leupeptin), and again centrifuged. The tissue was then resuspended in incubation buffer (1ml per 4 mg pancreas) and incubated for 15 minutes at room temperature, then 250 μl was added to each assay tube to commence the assay. The assay tubes contained incubation buffer consisting of 50 mM HEPES (pH 7.4), 0.5% BSA, protease inhibitors, 2 mM $Mncl_2$, 1 μM somatostatin, and concentrations of $^{125}$I-GRP and peptides as needed in a final volume of 500 μl. The assay was allowed to proceed to equilibrium for 90 minutes at room temperature. After this time, the contents of each tube was rapidly filtered over Whatman GF/B filters presoaked in 0.1% polyethyleneimine and the filters were rapidly washed three times with ice-cold 50 mM HEPES (pH 7.4). Filter-bound radioactivity was quantitated in a gamma counter. Competition of iodinated GRP binding by test compounds or standards was expressed as a percentage of $^{125}$I-GRP binding in the absence of peptide. Affinity and maximal binding were calculated with Ligand (Biosoft, Cambridge, UK) (Figure 1 and Figure 2).

EXAMPLE 4

ANTAGONISM OF THE BOMBESIN RECEPTOR ($Phe_{13}$ Ψ[$CH_2S$]$Leu_{14}$ BOMBESIN AS DEMONSTRATED BY THE EFFECT ON PHOSPHATIDYLINOSITOL TURNOVER

Pancreata from mice were pooled and chopped at 350 μm with a tissue chopper. The chopped tissue was washed twice with Krebs-Hepes buffer, then incubated for 30 minutes in 37°C Krebs-Hepes buffer with fresh buffer after 15 minutes. The tissue was then incubated in this buffer containing 100-200 μCi of $^3$H-inositol at 37°C for 1 hour. The tissue was then washed twice and incubated for another 30 minutes in Krebs-Hepes buffer (containing 10 mM $Li^+$) at 37°C with fresh buffer change after 15 minutes. Portions of the tissue mass (approximately 10 mg per assay tube) were then placed in $Li^+$ buffer with protease inhibitors, (40 μg/ml bacitracin,4 μg/ml leupeptin, 4 μg/ml chymostatin, 20 μg/ml thiorphan), 0.1% BSA, and then 0.1-1000 nM peptide was added in 25 μl in a final volume of 250 μl. To measure antagonism portions of the tissue in Li+ buffer were pretreated with 1 uM of the potential antagonist for 5 minutes at 25°C before the addition of agonist (GRP). After 60 minutes at room temperature, the phosphatidylinositol turnover was terminated by the addition of 940 μl chloroform:methanol (1:2), followed by 310 μl chloroform, followed by 310 μl water. Each tube was then vortexed for 5 seconds and then centrifuged at 2500 x g for 8 minutes to separate the phases. 900 μl of the top (aqueous) phase was then mixed with 2.1 ml water and loaded onto a 0.5 ml Biorad AG-1X8 (formate) ion exchange

column. 50 µl of the bottom phase (chloroform) was withdrawn from each tube and placed in a counting vial, dried, and counted in scintillation fluid. The material on the columns was washed in order with: 1) 10 ml of water 2) 5 ml of 5 mM disodium tetraborate/60 mM sodium formate 3) 10 ml of 1 M ammonium formate in 0.1 M formic acid. The final (third) wash was collected and one ml was mixed with 10 ml of Bio-Safe scintillant and counted. The ratio of these counts (total inositol phosphates) to the corresponding organic phase counts was then calculated for each sample. The ratios in the presence of test compound and/or standards were then compared to the ratios for control tubes (i.e., no stimulating agonist). Dose-response lines were constructed and the abilities of test compounds to stimulate or to inhibit GRP induced phosphatidylinositol turnover were determined by graphical analysis or with the aid of a computer program and are illustrated in Figures 3 and 4.

Figure 1 illustrates that $^{125}$I-GRP binds to a single class of sites the bombesin/GRP receptor on murine pancreatic membranes (Example 1). Binding of 25-1600 pM $^{125}$I-GRP was assayed in triplicate, then analyzed and plotted with LIGAND. The best computer fit of these data is 19 pM receptor per sample (~100 fmol receptor per mg membrane protein) with a Kd of 47 pM. The abscissa (x-axis) indicates the concentration of $^{125}$I-GRP bound to the receptor. The ordinate (y-axis) indicates the concentration of $^{125}$I-GRP bound to the receptor divided by the concentration of $^{125}$I-GRP that is free (not bound). The straight line is indicative of a single class of sites; that is, $^{125}$I-GRP binds to each of its receptors with the same affinity. Other experiments using 25-3200 pM $^{125}$I-GRP or 10 pit $^{125}$I-GRP ± 8-500 pM GRP also indicated a similar Kd. This shows that binding to the receptor for bombesin/GRP can be measured by competition for $^{125}$I-GRP binding to murine pancreatic membranes.

Figure 2 illustrates the ability of bombesin analogs to bind to the GRP receptor as demonstrated by the ability of these peptides to compete for binding of $^{125}$I-GRP to murine pancreatic membranes (Example 1). Binding was assayed in triplicate using 0.1 nM$^{125}$I-GRP and the indicated concentrations of peptide. The abscissa (x-axis) logarithmically indicates the concentration of agonists or antagonist of the GRP receptor. The ordinate (y-axis) indicates the observed binding for each tested peptide measured as a percentage of maximal $^{125}$I-GRP binding (no peptide present). LIGAND analysis of this and other experiments determined that bombesin and ($Phe_{13}Leu_{14}$) bombesin both have a Kd of 0.1 nM and that ($Phe_{13}[CH_2S]Leu_{14}$)bombesin has a $K_d$ of 3 nM.

-o-bombesin

-□- ($Phe_{13}Leu_{14}$)bombesin

-Δ-( $Phe_{13}(CH_2S)Leu_{14}$) bombesin

Figure 3 illustrates the ability of GRP, but not ($Phe_{13}[CH_2S]Leu_{14}$)bombesin, to stimulate phosphatidylinositol (PI) turnover in a dose-dependent manner (Example 2). The abscissa (x-axis) logarithmically indicates the concentration of peptide present. The ordinate (y-axis) indicates the observed PI turnover as a percentage of control. Values are mean ± standard error of triplicate determinations; where not shown, the errors are ≤the size of the data points. The lack of effect on PI turnover by ($Phe_{13}[CH_2S]Leu_{14}$)bombesin at concentrations 3 orders of magnitude greater than an amount of GRP which results in near maximal stimulation indicate that this peptide has no agonist activity.

-o-GRP

-Δ-($Phe_{13}[CH_2S)Leu_{14}$)bombesin

Figure 4 illustrates the ability of ($Phe_{13}[CH_2s]Leu_{14}$)bombesin to antagonize GRP-induced PI turnover in murine pancreas (Example 2). The abscissa (x-axis) logarithmically indicates the concentration of peptide present. The ordinate (y-axis) indicates the observed PI turnover as a percentage of control. Preincubation for 30 minutes with 1000 nM ($Phe_{13}$ $[CH_2S]Leu_{14}$)bombesin reduces the stimulation of PI turnover by 1-1000 nM GRP compared to GRP alone. Data are the mean ± standard error of triplicate determinations; where not shown, the errors are ≤ the size of the data points. The ability of ($Phe_{13}[CH_2S]Leu_{14}$) bombesin to inhibit GRP actions demonstrates that it is a true antagonist.

-o-GRP alone

-Δ-GRP after ($Phe_{13}[CH_2S]Leu_{14}$)bombesin

Figure 5: Reverse phase HPLC has been used to separate methylene sulfoxide isomers by employing a C18 stationary phase (Vydac analytical column; 4.6mm ID x 25cm, 5 micron C18). The mobile phase was pumped at a flow rate of 2 ml/minute with a linear gradient of 25% to 75% acetonitrile in water containing 0.1% trifluoroacetic acid over 25 minutes. $Phe\Psi[CH_2S(O)]^{I}$Leu Litorin and $Phe\Psi[CH_2S(O)]^{II}$Leu Litorin had retentions times of 5.40 minutes and 6.28 minutes respectively. The starting material of the reaction, $[Phe_8[CH_2S]Leu_9]$Litorin, in the HPLC system has a retention time of 10.92 minutes.

Table 1 compares the results of the earlier experiments (figures 1-4) for receptor affinity ($K_d$) and PI turnover for several of the bombesin analogs.

TABLE 1

| Affinity and Efficacy of Several Peptide Analogs | | | |
|---|---|---|---|
| | | PI Turnover | |
| Sequence | Kd (nM) | Agonist | Antag. |
| pQ Q R L G N Q W A V G H $F_{\Psi}$[$CH_2$S]L# | 3 | - | + |
| pQ Q W A V G H $F_{\Psi}$$CH_2$S]L# | 3 | | + |
| Acf Q W A V a H $F_{\Psi}$[$CH_2$S]L# | 10 | - | + |
| N Q W A V G H $F_{\Psi}$[$CH_2$S]L# | 50 | ND | ND |
| N Q W A V G H $F_{\Psi}$[$CH_2$S(O)]L# | 100 | ND | ND |
| pQ Q W A V G H $F_{\Psi}$ [$CH_2$S]Z# | 4 | ND | ND |
| pQ Q W A V G H $F_{\Psi}$[$CH_2$S(O)]$^{I}$L# | 2 | - | + |
| pQ Q W A V G H $F_{\Psi}$[$CH_2$S(O)]$^{II}$L# | 1 | - | + |

Kd was determined as in Example 1, PI turnover as in Example 2. "+" indicates activity, "-" indicates no activity, ND indicated that activity was not determined. Agonist activity is the ability to stimulate PI turnover, antagonists activity is the ability to block GRP-stimulted PI turnover. Ac, acetyl; pQ, pyroglutamic acid; Q, glutamine; A, alanine; R, arginine; N, aspartic acid; C, cysteine; E, glutamic acid; G, glycine; H, histidine; I, isoleucine; L, leucine; K, lysine; M, methionine; F, phenylalanine; P, proline; S, serine; T, threonine; W, tryptophan; Y, tyrosine; V, valine; upper case, L-amino acid; lower case, D-amino acid; Z, norleucine; #, amide bond; $\Psi$[$NH_2$S] has a $CH_2$S interamino acid linkage, $\Psi$[$CH_2$S(O)] has a $CH_2$S(O) interamino acid linkage, superscripts I and II refer to isomers of $CH_2$S(O);

**Claims**

**Claims for the following Contracting States : AT, BE, CH, DE, DK, FR, GB, IT, LI, LU, NL, SE**

**1.** A peptide derivative of the formula

$$X\text{-}N\text{-}A_1\text{-}A_2\text{-}A_3\text{-}A_4\text{-}A_5\text{-}A_6\text{-}A_7\text{-}A_8\text{-}A_9\text{-}A_{10}\text{-}A_{11}\text{-}A_{12}\text{-}A_{13}\text{-}\Psi\text{-}A_{14}\text{-}C\text{-}Y$$

| | |
|---|---|
| wherein X | is an amino terminal residue selected from hydrogen, one or two alkyl groups in from 1 to 10 carbon atoms, one or two acyl groups from 2 to 10 carbon atoms, carbobenzyloxy or t-butyloxy carbonyl; unless the amino terminal amino acid is a cyclic derivative and thereby X is omitted, |
| N | is a bond, unless the amino terminal amino acid is a cyclic derivative and thereby N is omitted, |
| $A_1$ | is pGlu or a bond |
| $A_2$ | is Gln or a bond |
| $A_3$ | is Arg or a bond |
| $A_4$ | is Leu or a bond |
| $A_5$ | is Gly or a bond |
| $A_6$ | is Asn, or D-Phe; |
| $A_7$ | is Gln or Eis; |
| $A_8$ | is Trp; |
| $A_9$ | is Ala; |
| $A_{10}$ | is Val; |
| $A_{11}$ | is Gly or D-Ala; |
| $A_{12}$ | is Eis; |
| $A_{13}$ | is Phe or Leu; |
| $\Psi$ | is a dipeptide determinant of $A_{13}$aa $\Psi$ $A_{14}$aa where $\Psi$ is [$CH_2$S], [$CH_2$S(O)], $\Psi$[$CH_2$S(O)]$^{I}$ or $\Psi$ [$CH_2$SO]$^{II}$ where in $A_{13}$aa and $A_{14}$aa designate the substituent amino acids; |
| $A_{14}$ | is Leu, Nle or Met; |
| C | is a bond; |
| Y | is a carboxy terminal residue selected from OE, ($C_1$-$C_8$) alkoxyester, amino, mono or di ($C_1$-$C_4$) alkyl amide ($C_1$-$C_4$)alkylanine, ($C_1$-$C_4$)thioalkylether, or pharmaceutically acceptable salt thereof. |

2. A peptide according to claim 1 wherein the peptide is pGlu-Gln-Arg-Leu-Gly-Asn-Gln-Trp-Ala-Val-Gly-His-PheΨ[$CH_2$S]Leu-$NH_2$ , Ac-D-Phe-Gln-Trp-Ala-Val-D-Ala-His-PheΨ[$CH_2$S]Leu-$NH_2$, Asn-Gln-Trp-Ala-Val-Gly-HisPheΨ[$CH_2$S]Leu-$NH_2$, Asn-Gln-Trp-Ala-Val-Gly-His-PheΨ[$CH_2$S(O)]Leu-$NH_2$,pGlu-Gln-Trp-Ala-Val-Gly-His-Phe-Ψ[$CH_2$S(O)]Leu-$NH_2$ , pGlu-Gln-Trp-Ala-Val-Gly-His-PheΨ[$CH_2$S]Nle-$NH_2$, pGlu-Gln-Trp-Ala-Val-Gly-His-PheΨ[$CH_2$S(O)]$^{I}$Leu-$NH_2$), pGlu-Gln-Trp-Ala-Val-Gly-His-PheΨ[$CH_2$S]Leu-$NH_2$ or pGlu-Gln-Trp-Ala-Val-Gly-His-PheΨ[$CH_2$S(O)]$^{II}$Leu-$NH_2$.

3. A process for preparing a peptide derivative according to claim 1 or 2 or a pharmaceutically acceptable salt thereof comprising the steps of:

   a) using a resin with a suitably bound C-terminal protected dipeptide from the group $A_{13}ΨA_{14}$, wherein Ψ is [$CH_2$S] and $A_{13}$ and $A_{14}$ designates the substituent amino acids;

   b) sequentially coupling the other alpha amino protected amino acids, $A_{12}$ through X, to achieve the protected amino acid sequence claimed;

   c) removing said protecting groups;

   d) purifying the desired peptide, or optionally forming the Ψ($CH_2$S(O)]$^{I}$, and Ψ($CH_2$SO]$^{II}$ isomers of said Ψ[$CH_2$S] peptide and then purifying the desired isomeric peptide.

4. Pharmaceutical composition comprising a peptide derivative or a pharmaceutically acceptable salt thereof according to claim 1 or 2 or a mixture thereof.

5. Pharmaceutical composition comprising a peptide derivative or a pharmaceutically acceptable salt thereof according to claim 1 to 2 or a mixture thereof for use in the treatment of cancers, including small cell lung and prostatic carcinomas.

6. Pharmaceutical composition comprising a peptide derivative or a pharmaceutically acceptable salt thereof according to claim 1 or 2 or a mixture thereof for use in the control of tumor tissue growth.

7. Pharmaceutical composition comprising a peptide derivative or a pharmaceutically acceptable salt thereof according to claim 1 or 2 or a mixture thereof for use in the treatment of peptic ulcers.

8. Pharmaceutical composition comprising a peptide derivative or a pharmaceutically acceptable salt thereof according to claim 1or 2 or a mixture thereof for use as bombesin/GRP antagonist.

9. Use of a peptide according to claim 1 or 2 or pharmaceutically acceptable salts thereof, optionally in combination with a pharmaceutically acceptable carrier, for the preparation of a pharmaceutical composition for the treatment of cancers, including small cell lung and prostatic carcinomas.

10. Use of a peptide according to claim 1 or 2 or pharmaceutically acceptable salts thereof, optionally in combination with a pharmaceutically acceptable carrier, for the preparation of a pharmaceutical composition for controlling the growth of tumor tissue.

11. Use of a peptide according to claim 1 or 2 or pharmaceutically acceptable salts thereof, optionally in combination with a pharmaceutically acceptable carrier, for the preparation of a pharmaceutical composition for the treatment of peptic ulcers.

12. Use of a peptide according to claim 1 or 2 or pharmaceutically acceptable salts thereof, optionally in combination with a pharmaceutically acceptable carrier, for the preparation of a pharmaceutical composition for use as a bombesin / GRP antagonist.

**Claims for the following Contracting State :ES**

1. A process for preparing a peptide derivative of the formula

$$X\text{-}N\text{-}A_1\text{-}A_2\text{-}A_3\text{-}A_4\text{-}A_5\text{-}A_6\text{-}A_7\text{-}A_8\text{-}A_9\text{-}A_{10}\text{-}A_{11}\text{-}A_{12}\text{-}A_{13}\text{-}\Psi\text{-}A_{14}\text{-}C\text{-}Y$$

wherein X is an amino terminal residue selected from hydrogen, one or two alkyl groups from 1 to 10 carbon atoms, one or two acyl groups from 2 to 10 carbon atoms, carbobenzyloxy or t-butyloxy carbonyl; unless the amino terminal amino acid is a cyclic derivative and thereby X is omitted,
N is a bond, unless the amino terminal amino acid is a cyclic derivative and thereby N is omitted,
$A_1$ is pGlu or a bond
$A_2$ is Gln or a bond
$A_3$ is Arg or a bond
$A_4$ is Leu or a bond
$A_5$ is Gly or a bond
$A_6$ is Asn or D-Phe;
$A_7$ is Gln or His;
$A_8$ is Trp;
$A_9$ is Ala;
$A_{10}$ is Val;
$A_{11}$ is Gly or D-Ala;
$A_{12}$ is Eis;
$A_{13}$ is Phe or Leu;
$\Psi$ is a dipeptide determinant of $A_{13}$aa $\Psi$ $A_{14}$aa where $\Psi$ is $[CH_2S]$, $[CH2_S(O)]$, $\Psi[CH_2S(O)]^I$ or $\Psi[CH_2S(O)]^{II}$ where in $A_{13}$aa and $A_{14}$aa designate the substituent amino acids:
$A_{14}$ is Leu, Nle or Met;
C is a bond;
Y is a carboxy terminal residue selected from OH, ($C_1$-$C_8$) alkoxyester, amino, mono or di ($C_1$-$C_4$) alkyl amide ($C_1$-$C_4$)alkylamine, ($C_1$-$C_4$)thioalkylether, or pharmaceutically acceptable salt thereof, comprising the steps of:

a) using a resin with a suitably bound C-terminal protected dipeptide from the group $A_{13}\Psi A_{14}$, wherein $\Psi$ is $[CH_2S]$ and $A_{13}$ and $A_{14}$ designates the substituent amino acids;
b) sequentially coupling the other alpha amino protected amino acids, $A_{12}$ through X, to achieve the protected amino acid sequence claimed;
c) removing said protecting groups;
d) purifying the desired peptide, or optionally forming the $\Psi[CH_2S(O)]^I$, and $\Psi[CH_2SO]^{II}$ isomers of said $\Psi[CH_2S]$ peptide and then purifying the desired isomeric peptide.

**2.** The process for preparing a peptide according to claim 1 wherein the peptide obtained. is pGlu-Gln-Arg-Leu-Gly-Asn-Gln-Trp-Ala-Val-Gly-His-Phe$\Psi[CH_2S]$Leu-$NH_2$, Ac-D-Phe-Gln-Trp-Ala-Val-D-Ala-His-Phe$\Psi[CH_2S]$Leu-$NH_2$,Asn-Gln-Trp-Ala-Val-Gly-His-Phe$\Psi[CH_2S]$Leu-$NH_2$, Asn-Gln-Trp-Ala-Val-Gly-His-Phe$\Psi[CH_2S(O)]$Leu-$NH_2$, pGlu-Gln-Trp-Ala-Val-Gly-Phe $\Psi[CH_2S(O)]$Leu- $NH_2$, pGlu-Gln-Trp-Ala-Val-Gly-His-Phe$\Psi$[Ch2S]Nle-$NH_2$, pGlu-Gln-Trp-Ala-Val-Gly-His-Phe$\Psi[CH_2S(O)]^I$Leu-$NH_2$] pGlu-Gln-Trp-Ala-Val-Gly-His-Phe$\Psi[CH_2S]$Leu-$NH_2$ or PGlu-Gln-Trp-Ala-Val-Gly-His-Phe$\Psi[CH_2S(O)]^{II}$Leu- $NH_2$.

## Patentansprüche

### Patentansprüche für folgende Vertragsstaaten: AT, BE, CH, DE, DK, FR, GB, IT, LI, LU, NL, SE

**1.** Peptidderivat mit der Formel

$$X\text{-}N\text{-}A_1\text{-}A_2\text{-}A_3\text{-}A_4\text{-}A_5\text{-}A_6\text{-}A_7\text{-}A_8\text{-}A_9\text{-}A_{10}\text{-}A_{11}\text{-}A_{12}\text{-}A_{13}\text{-}\Psi\text{-}A_{14}\text{-}C\text{-}Y$$

wobei

X ein aminoterminaler Rest ist, ausgewählt aus Wasserstoff, einem oder zwei Alkylresten mit 1 bis 10 Kohlenstoffatomen, einem oder zwei Acylresten mit 2 bis 10 Kohlenstoffatomen, Carbobenzyloxy- oder tButyloxycarbonylgruppen, es sei denn die aminoterminale Aminosäure ist ein cyclisches Derivat, wobei X entfällt,
N ist eine Bindung, es sei denn, die aminoterminale Aminosäure ist ein cyclisches Derivat, wobei N entfällt,

$A_1$ pGlu oder eine Bindung ist,
$A_2$ Gln oder eine Bindung ist,
$A_3$ Arg oder eine Bindung ist,
$A_4$ Leu oder eine Bindung ist,
$A_5$ Gly oder eine Bindung ist,
$A_6$ Asn oder D-Phe ist,
$A_7$ Gln oder His ist,
$A_8$ Trp ist,
$A_9$ Ala ist,
$A_{10}$ Val ist,
$A_{11}$ Gly oder D-Ala ist,
$A_{12}$ His ist,
$A_{13}$ Phe oder Leu ist,
$\Psi$ eine Dipeptiddeterminante von $A_{13}aa\Psi A_{14}aa$ ist, wobei $\Psi[CH_2S]$, $[CH_2S\ (O)]$, $\Psi[CH_2S\ (O)]^I$ oder $\Psi[CH_2SO]^{II}$ ist, wobei $A_{13}aa$ und $A_{14}aa$ die Substituenten-Aminosäuren bezeichnen,
$A_{14}$ Leu, Nle oder Met ist,
C eine Bindung ist,
Y ein carboxyterminaler Rest ist, ausgewählt aus OH, $(C_I$-$C_8)$ -Alkoxyester-, Amino-, Mono- oder Di$(C_1$-$C_4)$ -Alkylamid-$(C_1$-$C_4)$-Alkylamin-, $(C_I$-$C_4)$ -Thioalkylethergruppen oder pharmazeutisch verträglichen Salzen davon.

**2.** Peptid nach Anspruch 1, wobei das Peptid pGlu-Gln-Arg-Leu-Gly-Asn-Gln-Trp-Ala-Val-Gly-His-Phe$\Psi$ $[CH_2S]$ Leu-$NH_2$, Ac-D-Phe-Gln-Trp-Ala-Val-D-Ala-His-Phe$\Psi[CH_2S]$Leu-$NH_2$, Asn-Gln-Trp-Ala-Val-Gly-His-Phe$\Psi[CH_2S]$ Leu-$NH_2$, Asn-Gln-Trp-Ala-Val-Gly-His-Phe$\Psi[CH_2S(O)]$Leu-$NH_2$, pGlu-Gln-Trp-Ala-Val-Gly-His-Phe-$\Psi[CH_2S(O)]$ Leu-$NH_2$, pGlu-Gln-Trp-Ala-Val-Gly-His-Phe$\Psi[CH_2S]$ Nle-$NH_2$, pGlu-Gln-Trp-Ala-Val-Gly-His-Phe$\Psi$ $[CH_2S\ (O)]^I$Leu-$NH_2$, pGlu-Gln-Trp-Ala-Val-Gly-His-Phe$\Psi[CH_2S]$Leu-$NH_2$ oder pGlu-Gln-Trp-Ala-Val-Gly-His-Phe$\Psi$ $[CH_2S\ (O)]^{II}$LeU-$NH_2$ ist.

**3.** Verfahren zur Herstellung eines Peptidderivats nach Anspruch 1 oder 2 oder eines pharmazeutisch verträglichen Salzes davon, umfassend die folgenden Schritte:

(a) Verwendung eines Harzes mit einem geeignet gebundenen, C-terminal geschützten Dipeptid aus der Gruppe $A_{13}\Psi A_{14}$, wobei $\Psi$ $[CH_2S]$ bedeutet und $A_{13}$ und $A_{14}$ die Substituenten-Aminosäuren bezeichnen,
(b) schrittweises Koppeln der anderen $\alpha$-Amino-geschützten Aminosäuren $A_{12}$ bis X zum Erhalt der beanspruchten geschützten Aminosäuresequenz,
(c) Entfernen der Schutzgruppen,
(d) Reinigen des gewünschten Peptids oder gegebenenfalls Bildung von $\Psi[CH_2S\ (O)]^I$ und $\psi[CH_2SO]^{II}$-Isomeren des $\Psi[CH_2S]$-Peptids und im Anschluß daran Reinigen des gewünschten isomeren Peptids.

**4.** Arzneimittel, umfassend ein Peptidderivat oder ein pharmazeutisch verträgliches Salz davon nach Anspruch 1 oder 2 oder ein Gemisch davon.

**5.** Arzneimittel, umfassend ein Peptidderivat oder ein pharmazeutisch verträgliches Salz davon nach Anspruch 1 oder 2 oder ein Gemisch davon, zur Verwendung bei der Behandlung von Krebs, einschließlich kleinzelligen Lungenkarzinomen und Prostatakarzinomen.

**6.** Arzneimittel, umfassend ein Peptidderivat oder ein pharmazeutisch verträgliches Salz davon nach Anspruch 1 oder 2 oder ein Gemisch davon, zur Verwendung bei der Bekämpfung des Wachstums von Tumorgewebe.

**7.** Arzneimittel, umfassend ein Peptidderivat oder ein pharmazeutisch verträgliches Salz davon nach Anspruch 1 oder 2 oder ein Gemisch davon, zur Verwendung bei der Behandlung von Magengeschwüren.

**8.** Arzneimittel, umfassend ein Peptidderivat oder ein pharmazeutisch verträgliches Salz davon nach Anspruch 1 oder 2 oder ein Gemisch davon, zur Verwendung als Bombesin/GRP-Antagonist.

**9.** Verwendung eines Peptids nach Anspruch 1 oder 2 oder pharmazeutisch verträglicher Salze davon, gegebenenfalls in Kombination mit einem pharmazeutisch verträglichen Träger, zur Herstellung eines Arzneimittels zur Behandlung von Krebs, einschließlich kleinzelligen Lungenkarzinomen und Prostatakarzinomen.

**10.** Verwendung eines Peptids nach Anspruch 1 oder 2 oder pharmazeutisch verträglicher Salze davon, gegebenenfalls in Kombination mit einem pharmazeutisch verträglichen Träger, zur Herstellung eines Arzneimittels zur Bekämpfung des Wachstums von Tumorgewebe.

**11.** Verwendung eines Peptids nach Anspruch 1 oder 2 oder pharmazeutisch verträglicher Salze davon, gegebenenfalls in Kombination mit einem pharmazeutisch verträglichen Träger, zur Herstellung eines Arzneimittels zur Behandlung von Magengeschwüren.

**12.** Verwendung eines Peptids nach Anspruch 1 oder 2 oder pharmazeutisch verträglicher Salze davon, gegebenenfalls in Kombination mit einem pharmazeutisch verträglichen Träger, zur Herstellung eines Arzneimittels zur Verwendung als Bombesin/GRP-Antagonist.

**Patentansprüche für folgende Vertragsstaaten: ES**

**1.** Verfahren zur Herstellung eines Peptidderivats mit der Formel

$$X\text{-}N\text{-}A_1\text{-}A_2\text{-}A_3\text{-}A_4\text{-}A_5\text{-}A_6\text{-}A_7\text{-}A_8\text{-}A_9\text{-}A_{10}\text{-}A_{11}\text{-}A_{12}\text{-}A_{13}\text{-}\Psi\text{-}A_{14}C\text{-}Y$$

wobei

X ein aminoterminaler Rest ist, ausgewählt aus Wasserstoff, einem oder zwei Alkylresten mit 1 bis 10 Kohlenstoffatomen, einem oder zwei Acylresten mit 2 bis 10 Kohlenstoffatomen, Carbobenzyloxy- oder t-Butyloxycarbonylgruppen, es sei denn die aminoterminale Aminosäure ist ein cyclisches Derivat, wobei X entfällt,
N ist eine Bindung, es sei denn, die aminoterminale Aminosäure ist ein cyclisches Derivat, wobei N entfällt,
$A_1$ pGlu oder eine Bindung ist,
$A_2$ Gln oder eine Bindung ist,
$A_3$ Arg oder eine Bindung ist,
$A_4$ Leu oder eine Bindung ist,
$A_5$ Gly oder eine Bindung ist,
$A_6$ Asn oder D-Phe ist,
$A_7$ Gln oder His ist,
$A_8$ Trp ist,
$A_9$ Ala ist,
$A_{10}$ Val ist,
$A_{11}$ Gly oder D-Ala ist,
$A_{12}$ His ist,
$A_{13}$ Phe oder Leu ist,
$\Psi$ eine Dipeptiddeterminante von $A_{13}$aa$\Psi A_{14}$aa ist, wobei $\Psi[CH_2S]$, $[CH_2S(O)]$, $\Psi[CH_2S(O)]^I$ oder $\Psi[CH_2SO]^{II}$ ist, wobei $A_{13}$aa und $A_{14}$aa die Substituenten-Aminosäuren bezeichnen,
$A_{14}$ Leu, Nle oder Met ist,
C eine Bindung ist,
Y ein carboxyterminaler Rest ist, ausgewählt aus OH, $(C_1\text{-}C_8)$ -Alkoxyester-, Amino-, Mono- oder Di$(C_1\text{-}C_4)$ -Alkylamid-$(C_1\text{-}C_4)$-Alkylamin-, $(C_1\text{-}C_4)$-Thioalkylethergruppen oder pharmazeutisch verträglichen Salzen davon, umfassend die folgenden Schritte:

(a) Verwendung eines Harzes mit einem geeignet gebundenen, C-terminal geschützten Dipeptid aus der Gruppe $A_{13}\Psi A_{14}$, wobei $\Psi$ $[CH_2S]$ bedeutet und $A_{13}$ und $A_{14}$ die Substituenten-Aminosäuren bezeichnen,
(b) schrittweises Koppeln der anderen $\alpha$-Amino-geschützten Aminosäuren $A_{12}$ bis X zum Erhalt der beanspruchten geschützten Aminosäuresequenz,
(c) Entfernen der Schutzgruppen,
(d) Reinigen des gewünschten Peptids oder gegebenenfalls Bildung von $\Psi$ $[CH_2S(O)]^I$ und $\Psi$ $[CH_2SO]^{II}$-Isomeren des $\Psi[CH_2S]$-Peptids und im Anschluß daran Reinigen des gewünschten isomeren Peptids.

**2.** Verfahren zur Herstellung eines Peptids nach Anspruch 1, wobei das erhaltene Peptid pGlu-Gln-Arg-Leu-Gly-Asn-Gln-Trp-Ala-Val-Gly-His-Phe$\Psi[CH_2S]$Leu-$NH_2$, Ac-D-Phe-Gln-Trp-Ala-Val-D-Ala-His-Phe$\Psi[CH_2S]$Leu-$NH_2$, Asn-Gln-Trp-Ala-Val-Gly-His-Phe$\Psi[CH_2S]$Leu-$NH_2$,

Asn-Gln-Trp-Ala-Val-Gly-His-PheΨ[$CH_2S$ (O)]Leu-$NH_2$, pGlu-Gln-Trp-Ala-Val-Gly-His-Phe-Ψ[$CH_2S$ (O)] Leu-$NH_2$, pGlu-Gln-Trp-Ala-Val-Gly-His-PheΨ[$CH_2S$] Nle-$NH_2$, pGlu-Gln-Trp-Ala-Val-Gly-His-PheΨ[$CH_2S(O)]^I$Leu-$NH_2$, pGlu-Gln-Trp-Ala-Val-Gly-His-PheΨ[$CH_2S$]Leu-$NH_2$ oder pGlu-Gln-Trp-Ala-Val-Gly-His-PheΨ[$CH_2S(O)]^{II}$LeU-$NH_2$ ist.

## Revendications

### Revendications pour les Etats contractants suivants: AT, BE, CH, DE, DK, FR, GB, IT, LI, LU, NL, SE

**1.** Dérivé peptidique de formule :

$$X\text{-}N\text{-}A_1\text{-}A_2\text{-}A_3\text{-}A_4\text{-}A_5\text{-}A_6\text{-}A_7\text{-}A_8\text{-}A_9\text{-}A_{10}\text{-}A_{11}\text{-}A_{12}\text{-}A_{13}\text{-}\Psi\text{-}A_{14}\text{-}C\text{-}Y$$

| | |
|---|---|
| dans laquelle X | est un résidu amine terminal sélectionné parmi un atome d'hydrogène, un ou deux groupements alkyle ayant de 1 à 10 atomes de carbone, un ou deux groupements acyle ayant de 2 à10 atomes de carbone, un groupement carbobenzyloxy ou t-butyloxycarbonyle ; à moins que l'acide aminé amine terminal ne soit un dérivé cyclique et que X soit par conséquent omis, |
| N | est une liaison, à moins que l'acide aminé amine terminal ne soit un dérivé cyclique et que par conséquent N soit omis, |
| $A_1$ | est pGlu ou une liaison |
| $A_2$ | est Gln ou une liaison |
| $A_3$ | est Arg ou une liaison |
| $A_4$ | est Leu ou une liaison |
| $A_5$ | est Gly ou une liaison |
| $A_6$ | est Asn ou D-Phe ; |
| $A_7$ | est Gln ou His ; |
| $A_8$ | est Trp ; |
| $A_9$ | est Ala ; |
| $A_{10}$ | est Val ; |
| $A_{11}$ | est Gly ou D-Ala; |
| $A_{12}$ | est His ; |
| $A_{13}$ | est Phe ou Leu ; |
| Ψ | est un déterminant dipeptidique de $A_{13}$aaΨ$A_{14}$aa, étant entendu que Ψ est [$CH_2S$], [$CH_2S$ (O)], Ψ[$CH_2S(O)]^I$ ou Ψ[$CH_2SO]^{II}$ où $A_{13}$aa et $A_{14}$aa désignent les acides aminés substituants ; |
| $A_{14}$ | est Leu, Nle ou Met; |
| C | est une liaison ; |
| Y | est un résidu carboxyle terminal sélectionné parmi OH, un alcoxyester en $C_1$-$C_8$, une amine, un mono- ou di(alkyl en $C_1$-$C_4$)amide, une (alkyl en $C_1$-$C_4$)amine, un thio(alkyl en $C_1$-$C_4$)éther ou un sel pharmaceutiquement acceptable de ces derniers. |

**2.** Peptide selon la revendication 1, dans lequel le peptide est pGlu-Gln-Arg-Leu-Gly-Asn-Gln-Trp-Ala-Val-Gly-His-PheΨ[$CH_2S$]Leu-$NH_2$, Ac-D-Phe-Gln-Trp-Ala-Val-D-Ala-His-PheΨ[$CH_2S$]Leu-$NH_2$, Asn-Gln-Trp-Ala-Val-Gly-His-PheΨ[$CH_2S$]Leu-$NH_2$, Asn-Gln-Trp-Ala-Val-Gly-His-PheΨ[$CH_2S$(O)]Leu-$NH_2$, pGlu-Gln-Trp-Ala-Val-Gly-His-PheΨ[$CH_2S$(O)]Leu-$NH_2$, pGlu-Gln-Trp-Ala-Val-Gly-His-PheΨ[$CH_2S$]Leu-$NH_2$, pGlu-Gln-Trp-Ala-Val-Gly-His-PheΨ[$CH_2S(O)]^I$Leu-$NH_2$, pGlu-Gln-Trp-Ala-Val-Gly-His-PheΨ[$CH_2S$]Leu-$NH_2$ ou pGlu-Gln-Trp-Ala-Val-Gly-His-PheΨ[$CH_2S(O)]^{II}$Leu-$NH_2$.

**3.** Procédé de préparation d'un dérivé peptidique selon la revendication ou 2 ou d'un sel pharmaceutiquement acceptable de ce dernier, comprenant les étapes consistant à :

a) utiliser une résine portant un dipeptide protégé, C terminal, lié de façon appropriée, du groupe $A_{13}$Ψ$A_{14}$, étant entendu que Ψ est [$CH_2S$] et $A_{13}$ et $A_{14}$ désignent les acides aminés substituants ;
b) coupler séquentiellement les autres acides aminés protégés en α-amine, $A_{12}$ à X, pour obtenir la séquence d'acides aminés protégés revendiquée ;
c) séparer lesdits groupements protecteurs ;

d) purifier le peptide désiré ou former facultativement les isomères $\Psi[CH_2S(O)]^I$ et $\Psi[CH_2SO]^{II}$ dudit peptide $\Psi[CH_2S]$, puis purifier le peptide isomère désiré.

**4.** Composition pharmaceutique comprenant un dérivé peptidique ou un sel pharmaceutiquement acceptable de ce dernier selon les revendications 1 ou 2 ou un mélange de ces derniers.

**5.** Composition pharmaceutique comprenant un dérivé peptidique ou un sel pharmaceutiquement acceptable de ce dernier selon la revendication 1 ou 2 ou leur mélange à utiliser dans le traitement de cancers, y compris le cancer pulmonaire à petites cellules et le cancer prostatique.

**6.** Composition pharmaceutique comprenant un dérivé peptidique ou un sel pharmaceutiquement acceptable de ce dernier selon la revendication 1 ou 2 ou leur mélange à utiliser pour contrôler la croissance des tissus tumoraux.

**7.** Composition pharmaceutique comprenant un dérivé peptidique ou un sel pharmaceutiquement acceptable de ce dernier selon la revendication 1 ou 2 ou leur mélange à utiliser dans le traitement des ulcères peptiques.

**8.** Composition pharmaceutique comprenant un dérivé peptidique ou un sel pharmaceutiquement acceptable de ce dernier selon la revendication 1 ou 2 ou leur mélange à utiliser en tant qu'antagoniste de bombésine/GRP.

**9.** Utilisation d'un peptide selon la revendication 1 ou 2 ou de sels pharmaceutiquement acceptables de ce dernier, facultativement en association avec un véhicule pharmaceutiquement acceptable, pour la préparation d'une composition pharmaceutique destinée au traitement de cancers, y compris le cancer pulmonaire à petites cellules et le cancer prostatique.

**10.** Utilisation d'un peptide selon la revendication 1 ou 2 ou de sels pharmaceutiquement acceptables de ce dernier, facultativement en association avec un véhicule pharmaceutiquement acceptable, pour la préparation d'une composition pharmaceutique destinée à contrôler la croissance de tissus tumoraux.

**11.** Utilisation d'un peptide selon la revendication 1 ou 2 ou de sels pharmaceutiquement acceptables de ce dernier, facultativement en association avec un véhicule pharmaceutiquement acceptable, pour la préparation d'une composition pharmaceutique destinée au traitement d'ulcères peptiques.

**12.** Utilisation d'un peptide selon la revendication 1 ou 2 ou de sels pharmaceutiquement acceptables de ce dernier, facultativement en association avec un véhicule pharmaceutiquement acceptable, pour la préparation d'une composition pharmaceutique à utiliser comme antagoniste de bombésine/GRP.

**Revendications pour l'Etat contractant suivant: ES**

**1.** Procédé de préparation d'un dérivé peptidique de formule :

$$X\text{-}N\text{-}A_1\text{-}A_2\text{-}A_3\text{-}A_4\text{-}A_5\text{-}A_6\text{-}A_7\text{-}A_8\text{-}A_9\text{-}A_{10}\text{-}A_{11}\text{-}A_{12}\text{-}A_{13}\text{-}\Psi\text{-}A_{14}\text{-}C\text{-}Y$$

| | |
|---|---|
| dans laquelle X | est un résidu amine terminal sélectionné parmi un atome d'hydrogène, un ou deux groupements alkyle ayant de 1 à 10 atomes de carbone, un ou deux groupements acyle ayant de 2 à10 atomes de carbone, un groupement carbobenzyloxy ou t-butyloxycarbonyle; à moins que l'acide aminé amine terminal ne soit un dérivé cyclique et que X soit par conséquent omis, |
| N | est une liaison, à moins que l'acide aminé amine terminal ne soit un dérivé cyclique et que par conséquent N soit omis, |
| $A_1$ | est pGlu ou une liaison |
| $A_2$ | est Gln ou une liaison |
| $A_3$ | est Arg ou une liaison |
| $A_4$ | est Leu ou une liaison |
| $A_5$ | est Gly ou une liaison |
| $A_6$ | est Asn ou D-Phe ; |
| $A_7$ | est Gln ou His ; |
| $A_8$ | est Trp ; |
| $A_9$ | est Ala ; |

| | |
|---|---|
| $A_{10}$ | est Val; |
| $A_{11}$ | est Gly ou D-Ala ; |
| $A_{12}$ | est His ; |
| $A_{13}$ | est Phe ou Leu ; |
| $\Psi$ | est un déterminant dipeptidique de $A_{13}$aa$\Psi A_{14}$aa, étant entendu que $\Psi$ est $[CH_2S]$, $[CH_2S(O)]$, $\Psi[CH_2S(O)]^{I}$ ou $\Psi[CH_2O]^{II}$ où $A_{13}$aa et $A_{14}$aa désignent les acides aminés substituants ; |
| $A_{14}$ | est Leu, Nle ou Met ; |
| C | est une liaison ; |
| Y | est un résidu carboxyle terminal sélectionné parmi OH, un alcoxyester en $C_1$-$C_8$, une amine, un mono- ou di(alkyl en $C_1$-$C_4$)amide, une (alkyl en $C_1$-$C_4$)amine, un thio(alkyl en $C_1$-$C_4$)éther ou un sel pharmaceutiquement acceptable de ces derniers, comprenant les étapes consistant à : |

a) utiliser une résine portant un dipeptide protégé, C terminal, lié de façon appropriée, du groupe $A_{13}\Psi SA_{14}$, étant entendu que $\Psi$ est $[CH_2S]$ et $A_{13}$ et $A_{14}$ désignent les acides aminés substituants ;
b) coupler séquentiellement les autres acides aminés protégés en $\alpha$-amine, $A_{12}$ par X, pour obtenir la séquence d'acides aminés protégés revendiquée ;
c) séparer lesdits groupements protecteurs ;
d) purifier le peptide désiré ou former facultativement les isomères $\Psi[CH_2S(O)]^{I}$ et $\Psi[CH_2SO]^{II}$ dudit peptide $\Psi[CH_2S$, puis purifier le peptide isomère désiré.

**2.** Procédé de préparation d'un peptide selon la revendication 1, dans lequel, le peptide obtenu est pGlu-Gln-Arg-Leu-Gly-Asn-Gln-Trp-Ala-Val-Gly-His-Phe$\Psi[CH_2S]$Leu-$NH_2$, Ac-D-Phe-Gln-Trp-Ala-Val-D-Ala-His-Phe$\Psi[CH_2S]$Leu-$NH_2$, Asn-Gln-Trp-Ala-Val-Gly-His-Phe$\Psi[CH_2S]$Leu-$NH_2$, Asn-Gln-Trp-Ala-Val-Gly-His-Phe$\Psi[CH_2S(O)]$Leu-$NH_2$, pGlu-Gln-Trp-Ala-Val-Gly-His-Phe$\Psi[CH_2S(O)]$Leu-$NH_2$, pGlu-Gln-Trp-Ala-Val-Gly-His-Phe$\Psi[CH_2S]$Nle-$NH_2$, pGlu-Gln-Trp-Ala-Val-Gly-His-Phe$\Psi[CH_2S(O)]^{I}$Leu-$NH_2$, pGlu-Gln-Trp-Ala-Val-Gly-His-Phe$\Psi[CH_2S]$Leu-$NH_2$ ou pGlu-Gln-Trp-Ala-Val-Gly-His-Phe$\Psi[CH_2S(O)]^{II}$Leu-$NH_2$.

Figure 1/5

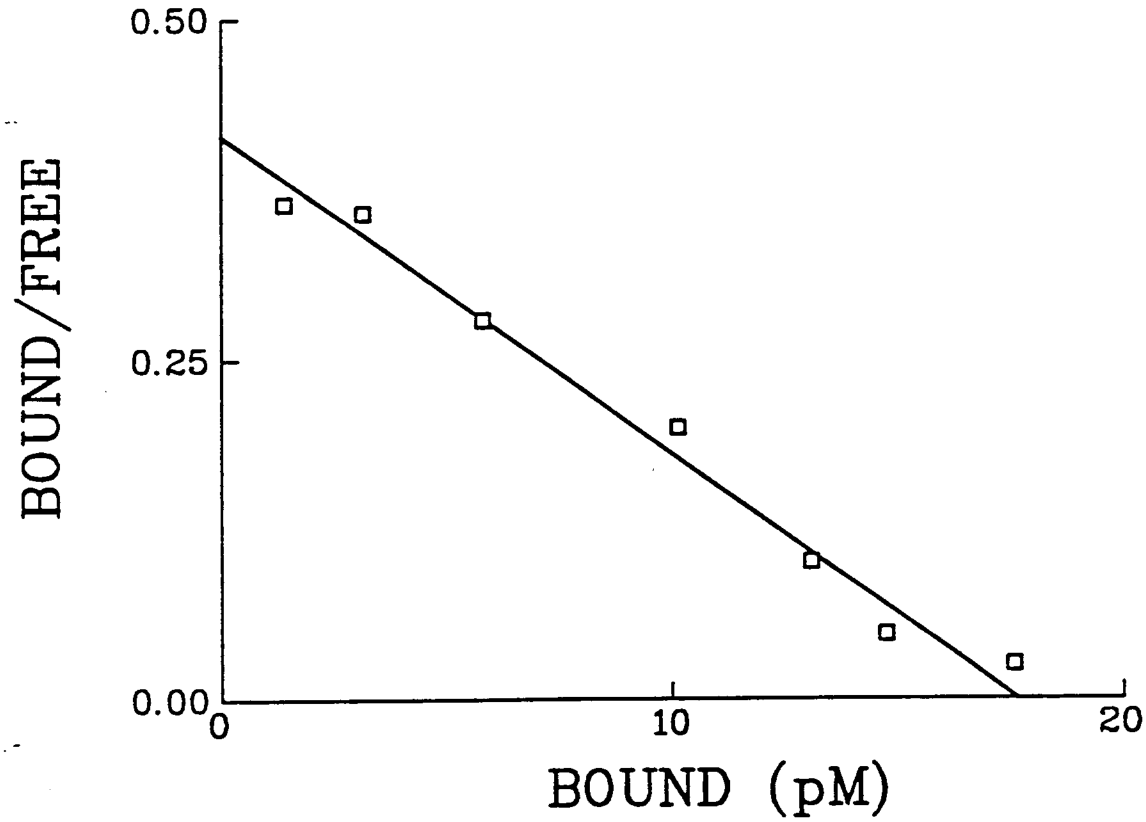

Figure 2/5

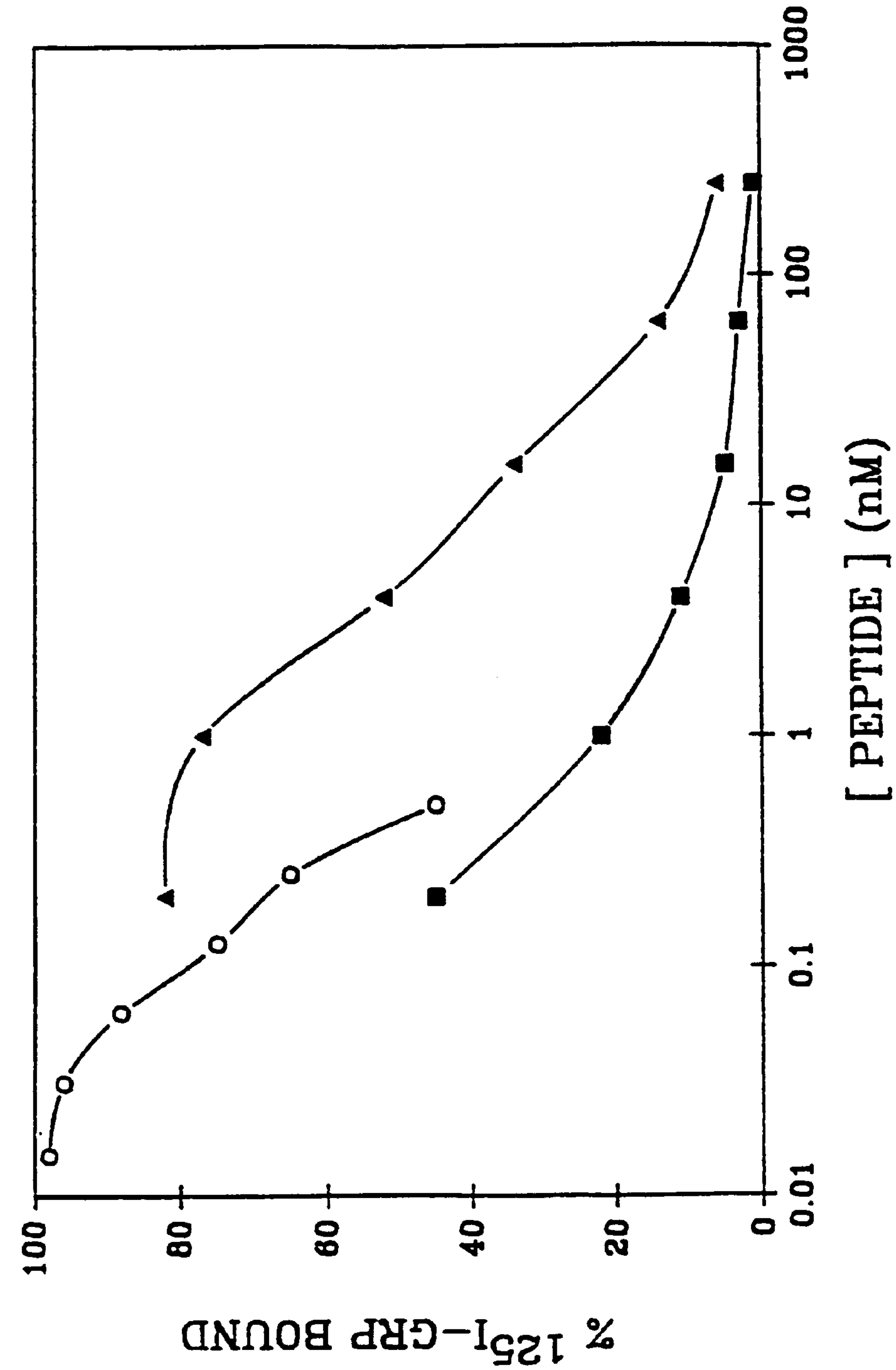

Figure 3/5

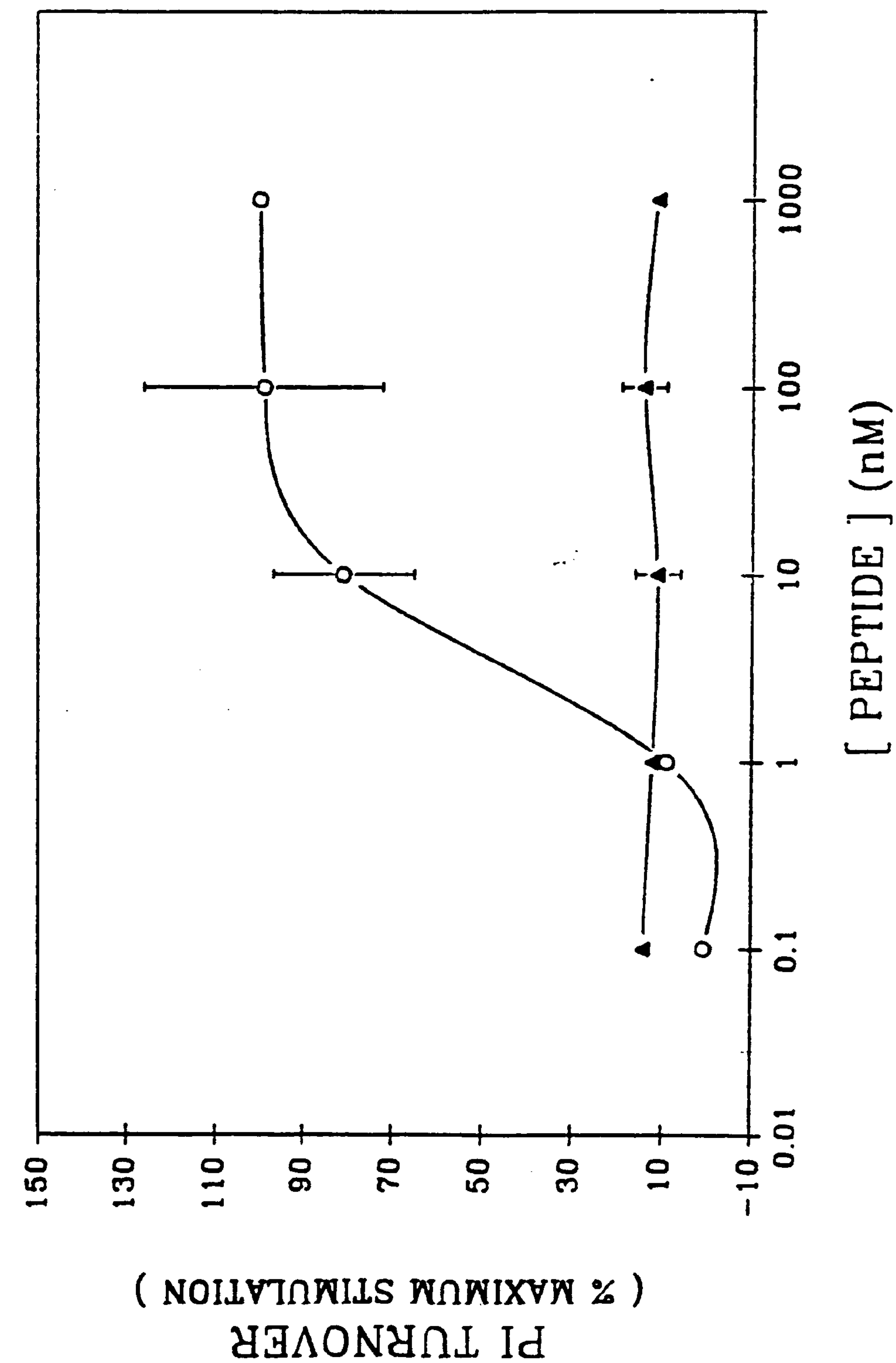
PI TURNOVER
( % MAXIMUM STIMULATION )
150
130
110
90
70
50
30
10
-10
0.01
0.1
1
10
100
1000
[ PEPTIDE ] (nM)

Figure 4 /5

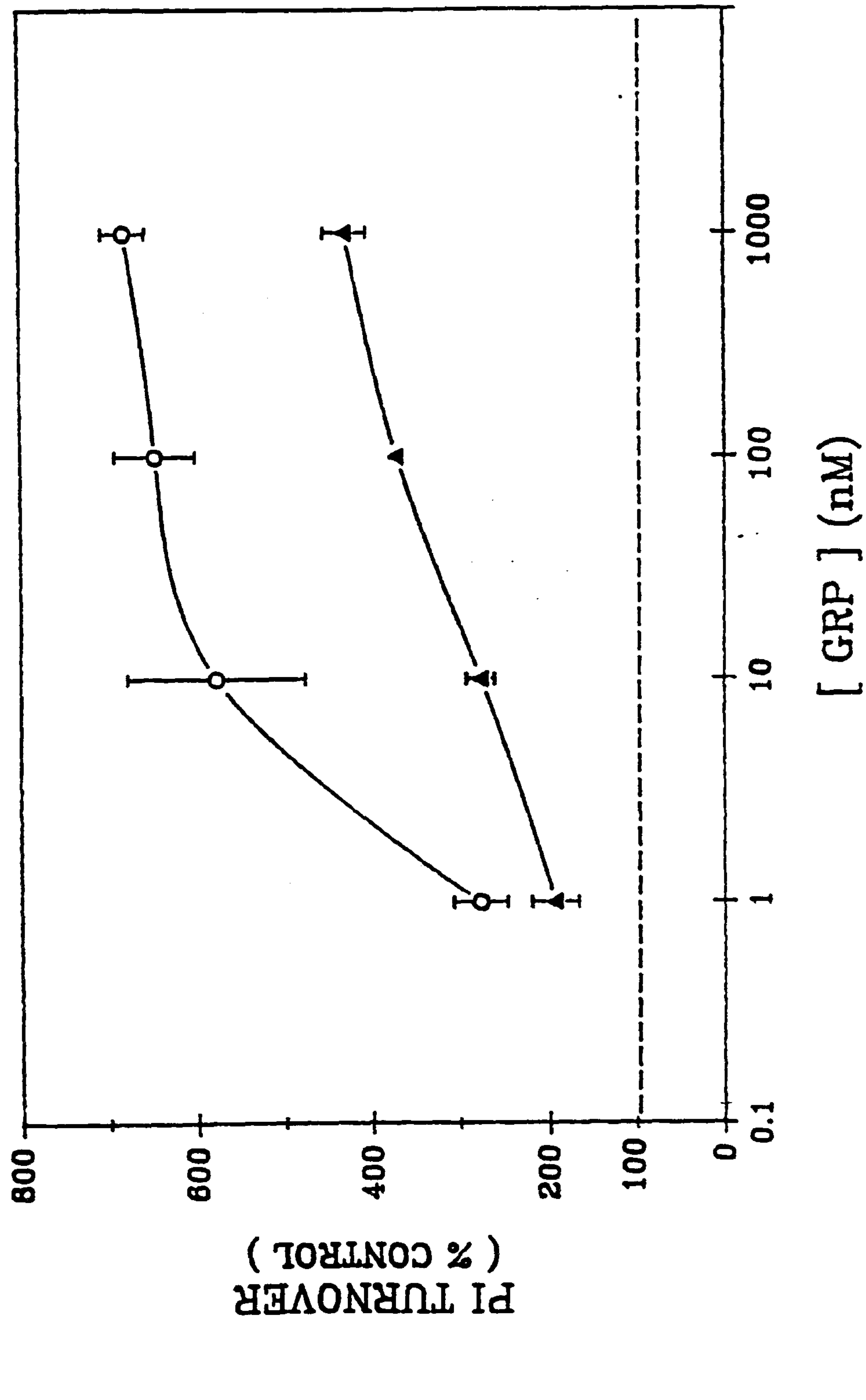
PI TURNOVER
( % CONTROL )
800
600
400
200
100
0
0.1
1
10
100
1000
[ GRP ] (nM)

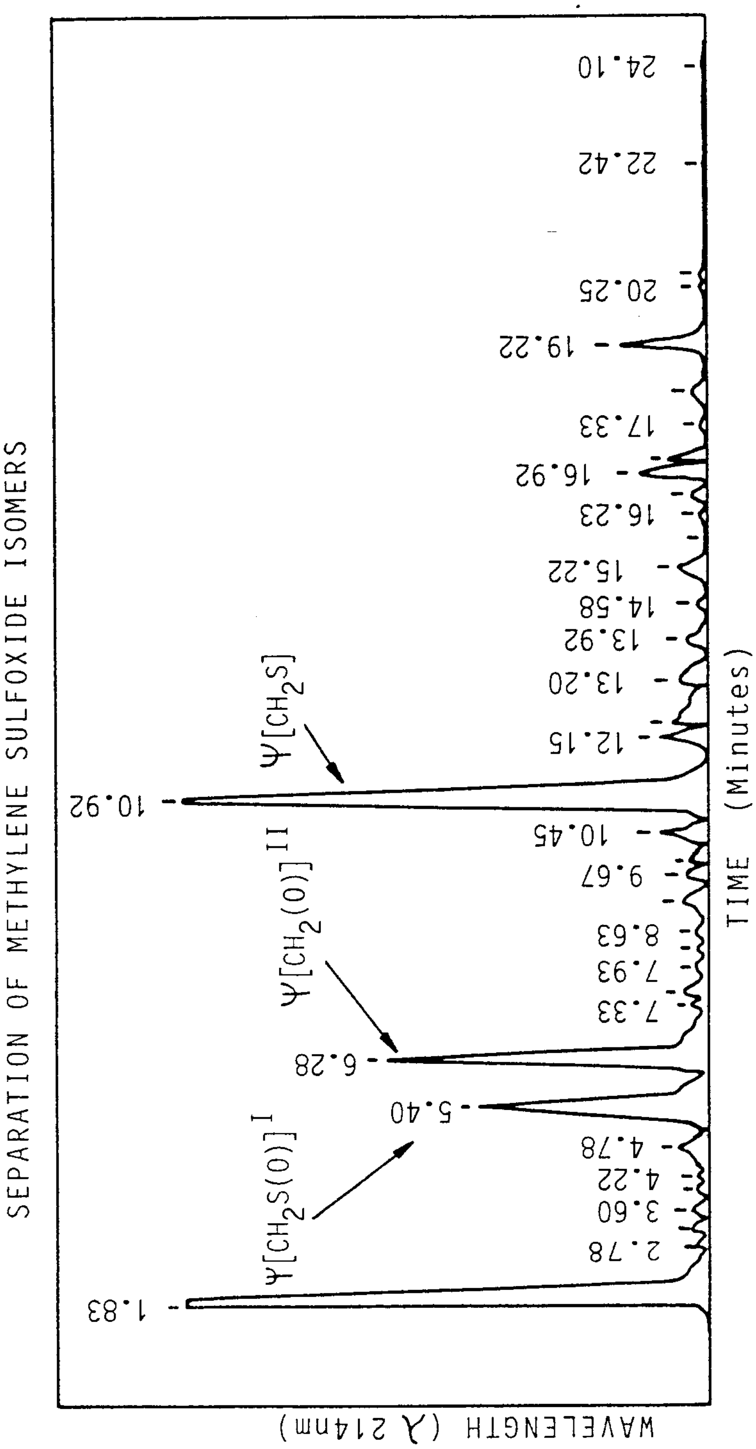
SEPARATION OF METHYLENE SULFOXIDE ISOMERS
TIME (Minutes)
WAVELENGTH (λ 214nm)
Ψ[CH2S(O)]I
Ψ[CH2(O)]II
Ψ[CH2S]
1.83
2.78
3.60
4.22
4.78
5.40
6.28
7.33
7.93
8.63
9.67
10.45
10.92
12.15
13.20
13.92
14.58
15.22
16.23
16.92
17.33
19.22
20.25
22.42
24.10

Fig. 5